# EUROPEAN PATENT APPLICATION

(11) **EP 1 229 019 A1**
(43) Date of publication of application: **07.08.2002**
(21) Application number: 00974856.7
(22) Date of filing: 09.11.2000
(51) Int. Cl.: C07C 221/00, C07C 225/20, C07D 215/38

(54) **PROCESS FOR THE PRODUCTION OF OPTICALLY ACTIVE CYCLIC ENAMINONE DERIVATIVES**

(30) Priority: 10.11.1999 JP 31999199
(71) Applicant: Takeda Chemical Industries, Ltd., Osaka-shi, Osaka 541-0045 (JP)
(72) Inventor: MIKI, Shokyo, Toyonaka-shi, Osaka 561-0846 (JP); SUIDE, Haruo, Toyono-gun, Osaka 563-0211 (JP); TAWADA, Hiroyuki, Takatsuki-shi, Osaka 569-1032 (JP); IWANO, Norio, Toyono-gun, Osaka 563-0102 (JP); AOKI, Isao, Kawanishi-shi, Hyogo 666-0111 (JP); ADACHI, Mari, Nishi-ku, Kobe-shi, Hyogo 651-2216 (JP)
(74) Representative: Lewin, John Harvey
(86) International application number: JP0007876
(87) International publication number: WO0134557

(57) **Abstract**

A process for the production of optically active cyclic enaminone derivatives characterized by aminating one of the carbonyl groups of a cyclic 1,3-diketone derivative having a symmetry plane to obtain an optically isomeric mixture of chiral cyclic enaminone derivatives and subjecting this mixture to optical resolution.

## Description

### FIELD OF THE INVENTION

The present invention relates to a process for the production of optically active cyclic enaminone derivatives, which are synthetic raw materials for producing pharmaceuticals, agrochemicals and the like.

### BACKGROUND OF THE INVENTION

In the case of stereo-selectively synthesizing the desired pharmaceuticals or agrochemicals, it is wasteful to carry out resolution of isomers in a step near the final step since the yield of the isomer resolution is theoretically not more than 50%. Accordingly, to obtain an optically active intermediate in an initial stage of the production process and reach the desired final compound while maintaining this stereo-structure is preferable from a viewpoint of synthetic scheme.

Further, as a method for optically resolving chiral compounds having an amino group, there has been known a method for isolation of a salt formed by adding an optically active acid.

Enaminone derivatives are useful compounds as synthetic raw materials for producing pharmaceuticals and agrochemicals. Likewise, in the case of stereo selectively producing pharmaceuticals and agrochemicals using the enaminone derivatives as the synthetic raw material compounds, if optically active derivatives are used from the beginning, the decrease in yield owing to resolution of optical isomers in later steps can be avoided and the production cost can be remarkably reduced.

However, enaminone derivatives are liable to cause substitution or exchange of the amino group under acidic conditions due to the partial structure of the enaminone , and unstable as compared with common organic bases such as alkylamines, monoarylamines, heterocyclic bases and alkaloids. For such reasons, it has scarcely been reported that enaminone derivatives can optically be resolved with an optically active acid, and the possibility of the resolution has not sufficiently been investigated heretofore in the prior art.

### DISCLOSURE OF THE INVENTION

In view of the above circumstances, the present inventors have studied a method capable of optical resolution of enaminone derivatives without making the structure unstable. As a result, the present inventors have unexpectedly found that an optical isomeric mixture of cyclic enaminone derivatives (a racemic mixture: this does not mean "conglomerate" but simply means (±)-compounds or an equivalent mixture without defining a particular specific existing state) can optically resolved at a high yield by treatment with a specified optically active acid. Further, it is found that, if a cyclic 1,3-diketone derivative having a symmetry plane is used as a precursor of the desired optically active cyclic enaminone derivative, after one desired optical isomer is resolved from a racemic mixture of chiral cyclic enaminone derivatives produced by using said compound as the raw material, the other optical isomer can be turned back to the raw material, the cyclic 1,3-diketone derivative having a symmetry plane, again. Thus, the present invention has been completed by confirming that the combination of these methods makes possible to be converted to one desired optically active isomer of the cyclic enaminone derivatives without wasting the raw material, the cyclic 1,3-diketone derivative having a symmetry plane.

That is, the present invention provides:
(1) A process for producing an optically active cyclic enaminone derivative comprising optically resolving an optical isomeric mixture of cyclic enaminone derivatives using an optical resolution agent with a high acidity;
(2) A process for producing an optically active cyclic enaminone derivative comprising aminating one carbonyl group of the diketone of a cyclic 1,3-diketone derivative having a symmetric plane to obtain an optical isomeric mixture of chiral cyclic enaminone derivatives and subjecting the mixture to optical resolution;
(3) A process for producing an optically active cyclic enaminone derivative comprising using a cyclic 1,3-diketone derivative having asymmetry plane as a raw material, aminating one carbonyl group of the diketone of the derivative to obtain an optical isomeric mixture of chiral cyclic enaminone derivatives, subjecting the mixture to optical resolution to obtain one optically active cyclic enaminone derivative, and hydrolyzing the other optically active cyclic enaminone derivative to recover the raw material, the cyclic 1,3-diketone derivative having a symmetry plane;
(4) The process according to the above (2) or (3), wherein the cyclic 1,3-diketone derivative having a symmetry plane has a 5- to 7-membered ring structure;
(5) The process according to the above (2) or (3), wherein the cyclic 1,3-diketone derivative having a symmetry plane is represented by the following formula (I): 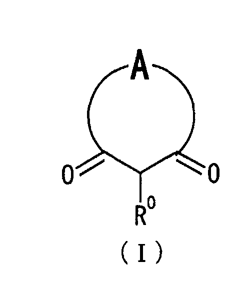 and the partial structure (A) of the formula (I): 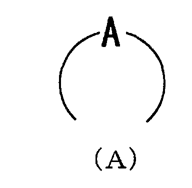 is represented by one of the following formulas (A-1) to (A-3): 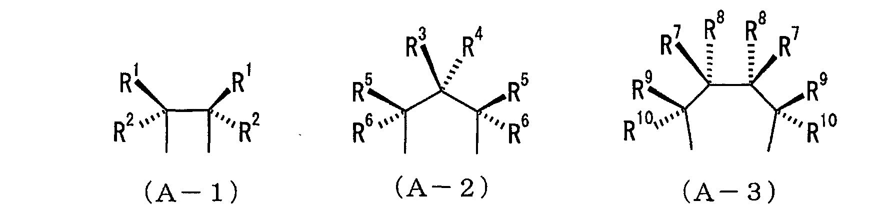 wherein R⁰ to R¹⁰ independently denote hydrogen atom or a substituent (provided that R¹ and R² do not denote the same group; R³ and R⁴ as well as R⁵ and R⁶, respectively, do not denote the same group simultaneously; and R⁷ and R⁸ as well as R⁹ and R¹⁰, respectively, do not denote the same group simultaneously);
(6) The process according to the above (5), wherein the partial structure (A) is represented by the following formula (A-1): 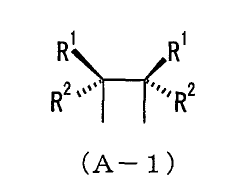 wherein R¹ and R² are as defined above;
(7) The process according to the above (5), wherein said partial structure (A) is represented by the following formula (A-2) : 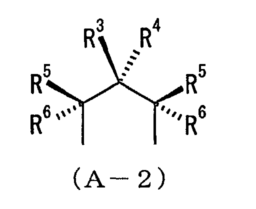 wherein R³, R⁴, R⁵ and R⁶ are as defined above;
(8) The process according to the above (7), wherein R³ and R⁴ of said compound represented by the formula (A-2) of said partial structure (A) independently denote hydrogen atom or an optionally substituted 5- or 6-membered aromatic homocyclic or heterocyclic group, respectively; R⁵ and R⁶ independently denote hydrogen atom, a halogen atom, an optionally substituted straight or branched chain aliphatic hydrocarbon group, an optionally substituted hydroxy group, or an optionally substituted mercapto group (provided that R³ and R⁴ as well as R⁵ and R⁶, respectively, do not denote the same group simultaneously);
(9) The process according to the above (7), wherein R³ and R⁴ of said compound represented by the formula (A-2) of said partial structure (A) independently denote hydrogen atom or an optionally substituted 5- or 6-membered aromatic homocyclic or heterocyclic group, respectively, (provided that R² and R³ do not denote the same group); R⁵ and R⁶ denote hydrogen atom;
(10) The process according to the above (5), wherein said partial structure (A) is represented by the following formula (A-3): 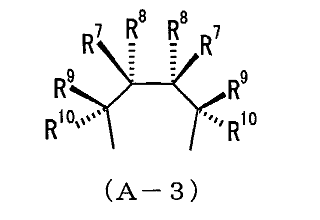 wherein R⁷ to R¹⁰ are as defined above;
(11) The process according to the above (2) or (3), wherein the optical resolution is carried out using an optical resolution agent with a high acidity;
(12) The process according to the above (1) or (11), wherein the optical resolution agent is an optically active sulfonic acid derivative or sulfamic acid derivative;
(13) The process according to the above (1) or (11), wherein the optical resolution agent is an optically active phosphoric acid derivative;
(14) An optically active cyclic enaminone derivative represented by the following formula: 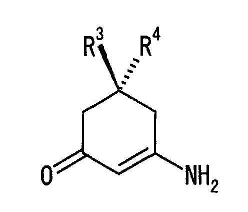 wherein R³ and R⁴ independently denote hydrogen atom or an optionally substituted 5- or 6-membered aromatic homocyclic or heterocyclic group, respectively (provided that R³ and R⁴ do not denote the same group);
(15) A process for producing (-)-3-amino-5-(5-fluoro-2-methylphenyl)-2-cyclohexenon-1-one comprising optically resolving (±)-3-amino-5-(5-fluoro-2-methylphenyl)-2-cyclohexenon-1-one using (-)-4-(mono- or di-chloro or methoxyphenyl)-2-hydroxy-5,5-dimethyl-1,3,2-dioxaphosphorinane-2-oxide; and
(16) A process for producing (5E, 7S)-[7-(5-fluoro-2-methylphenyl)-4-methyl-7,8-dihydro-5(6H)-quinolinylideneamino]guanidine or its salt comprising reacting (-)-7-(5-fluoro-2-methylphenyl)-4-methyl-7,8-dihydroquinolin-5(6H)-one, which is obtained by reacting 1,1-dimethoxy-3-butanone with (-)-3-amino-5-(5-fluoro-2-methylphenyl)-2-cyclohexenon-1-one obtained by optically resolving (±)-3-amino-5-(5-fluoro-2-methylphenyl)-2-cyclohexenon-1-one using (-)-4-(mono- or di-chloro or methoxyphenyl)-2-hydroxy-5,5-dimethyl-1,3,2-dioxaphosphorinane-2-oxide, with aminoguanidine or its salt.

In the present specification, the term, "cyclic 1,3-diketone derivative having a symmetry plane" means such compound that, when the cyclic 1,3-diketone derivative is divided into two divided portions, respective divided portions have planes to be mirror images to each other and any compounds satisfying this condition may be used for the production process of the present invention, but, among these compounds, examples of the preferred compounds to be used for the production process of the present invention include those represented by the following formula (I): 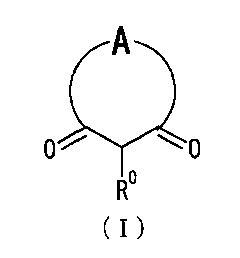 and in the formula (I), the partial structure (A): 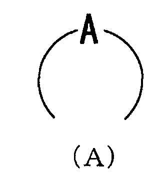 is represented by one of the following formulas (A-1) to (A-3): 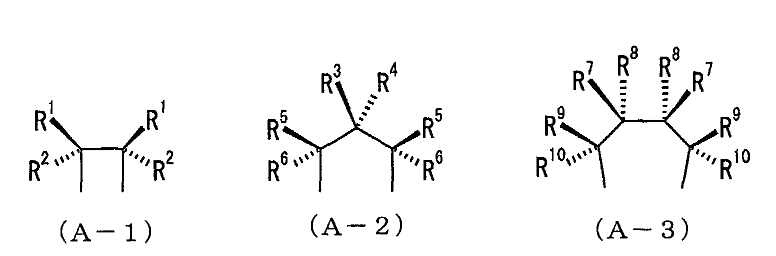 wherein R⁰ to R¹⁰ are as defined above.

"The substituents" of R⁰ to R¹⁰ are a halogen atom, an optionally substituted straight or branched chain aliphatic hydrocarbon group, an optionally substituted hydroxy group, an optionally substituted mercapto group, an optionally substituted amino group, an optionally substituted imidoyl group, an optionally substituted amidino group, nitro group, cyano group, optionally esterified or amidated carboxyl group, an optionally substituted sulfonyl, an acyl group derived from a carboxylic acid, an acyl group derived from a sulfonic acid, an acyl group derived from a sulfinic acid, an optionally substituted aromatic or non-aromatic homocyclic group, an optionally substituted aromatic or non-aromatic heterocyclic group and the like.

Examples of the "halogen atom" used herein include fluorine, chlorine, bromine, iodine and the like, and preferably are chlorine and bromine and the like.

Examples of the "straight chain or branched chain aliphatic hydrocarbon group" include an alkyl group, an alkenyl group, an alkynyl group and the like.

The alkyl group is, for example, C₁₋₁₀ alkyl group such as methyl, ethyl, n-propyl, isopropyl, n-butyl, isobutyl, sec-butyl, tert-butyl, n-pentyl, isopentyl, neopentyl, 1-methylpropyl, n-hexyl, isohexyl, 1,1-dimethylbutyl, 2,2-dimethylbutyl, 3,3-dimethylbutyl, 3,3-dimethylpropyl, 2-ethylbutyl, n-heptyl, 1-methylheptyl, 1-ethylhexyl, n-octyl, 1-methylheptyl, nonyl and the like, and preferably lower (C₁₋₆) alkyl.

The alkenyl group is, for example, C₂₋₆ alkenyl group such as vinyl, allyl, isopropenyl, 2-methylallyl, 1-propenyl, 2-methyl-1-propenyl, 1-butenyl, 2-butenyl, 3-butenyl, 2-ethyl-1-butenyl, 2-methyl-2-butenyl, 3-methyl-2-butenyl, 1-pentenyl, 2-pentenyl, 3-pentenyl, 4-pentenyl, 4-methyl-3-pentenyl, 1-hexenyl, 2-hexenyl, 3-hexenyl, 4-hexenyl, 5-hexenyl and the like.

The alkynyl group is, for example, C₂₋₆ alkynyl group such as ethynyl, 1-propynyl, 2-propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl, 5-hexynyl and the like.

These "straight chain or branched chain aliphatic hydrocarbon groups" may further be substituted at any possible position with 1 to 3 the same or different substituents selected from, for example, halogen atoms; optionally substituted hydroxy groups such as lower (C₁₋₆) alkoxy groups, C₃₋₆ cycloalkyloxy groups, aryloxy groups, etc.; optionally substituted mercapto groups such as lower (C₁₋₆) alkylthio groups, C₃₋₆ cycloalkylthio groups, arylthio groups, etc.; amino groups optionally substituted with one or two the same or different substituents selected from lower (C₁₋₆) alkyl, lower (C₁₋₆) alkoxy, C₃₋₆ cycloalkyl, hydroxy, carbamoyl, phenyl, phenyl-lower (C₁₋₆) alkyl, lower (C₁₋₆) alkanoyl, C₃₋₆ cycloalkyl-carbonyl, benzoyl, phenyl-C₂₋₆ alkanoyl, lower (C₁₋₆) alkoxy-carbonyl, phenoxy-carbonyl, phenyl-C₁₋₆ alkoxy-carbonyl, lower (C₁₋₆) alkylsulfinyl, C₃₋₆ cycloalkylsulfinyl, phenylsulfinyl, lower (C₁₋₆) alkylsulfonyl, C₃₋₆ cycloalkylsulfonyl, lower (C₁₋₆) alkoxysulfonyl, and phenylsulfonyl, etc., or optionally substituted amino groups such as cycloamino groups formed by two substituents bound to each other and the nitrogen atom; imidoyl and amidino groups optionally substituted with lower (C₁₋₆) alkyl, lower (C₁₋₆) alkoxy, C₃₋₆ cycloalkyl, aryl, etc.; nitro groups; cyano groups; optionally esterified carboxyl groups such as lower alkoxycarbonyl group, aryloxycarbonyl group, aralkyloxycarbonyl group, etc.; N-mono-substituted or N,N-di-substituted carbamoyl groups optionally substituted with one or two the same or different substituents selected from lower alkyl, cycloalkyl, aryl, aralkyl, and aromatic or non-aromatic homocyclic groups and aromatic or non-aromatic heterocyclic groups, described hereinafter; optionally substituted sulfonyl groups such as lower (C₁₋₆) alkylsulfonyl, C₃₋₆ cycloalkylsulfonyl, phenyl-C₁₋₆ alkylsulfonyl, lower (C₁₋₆) alkoxysulfonyl, C₃₋₆ cycloalkyloxysulfonyl, phenyl-C₁₋₆ alkoxysulfonyl, sulfamoyl, lower (C₁₋₆) alkylaminosulfonyl, C₃₋₆ cycloalkylaminosulfonyl, phenyl-C₁₋₆ alkylaminosulfonyl, cyclicaminosulfonyl, nitroxy-C₁₋₆ alkylaminosulfonyl, anilinosulfonyl, etc.; carboxylic acid-derived acyl groups such as lower (C₁₋₆) alkyl-carbonyl (alkanoyl), C₃₋₆ cycloalkyl-carbonyl, benzoyl, etc.; sulfonic acid-derived acyl groups such as lower (C₁₋₆) alkylsulfonyl, C₃₋₆ cycloalkylsulfonyl, phenylsulfonyl, etc.; sulfinic acid-derived acyl groups such as lower (C₁₋₆) alkylsulfinyl, C₃₋₆ cycloalkylsulfinyl, phenylsulfinyl, etc.; saturated or unsaturated alicyclic hydrocarbon groups such as cycloalkyl group, cycloalkenyl group, cycloalkanedienyl group, etc.; monocyclic or fused polycyclic aromatic hydrocarbon groups such as aryl group; 5- and 6-membered aromatic monocyclic heterocyclic groups; 8- to 12-membered aromatic fused heterocyclic groups; and the like.

As the "optionally substituted hydroxy group", in addition to free hydroxy group, there are, for example, (C₁₋₆) alkoxy groups (methoxy, ethoxy, propoxy, isopropoxy, butoxy, isobutoxy, sec-butoxy, tert-butoxy, pentyloxy, hexyloxy and the like), C₃₋₆ cycloalkyloxy groups (cyclopropyloxy, cyclobutyloxy, cyclopentyloxy, cyclohexyloxy and the like), aryloxy groups (phenyloxy, naphthyloxy and the like) and the like.

These substituted hydroxy groups may further be substituted at any possible position with 1 to 3 the same or different substituents selected from, for example, halogen atoms; straight chain or branched chain aliphatic hydrocarbon groups such as alkyl group, alkenyl group, alkynyl group, etc.; optionally substituted hydroxy groups such as lower (C₁₋₆) alkoxy groups, C₃₋₆ cycloalkyloxy groups, aryloxy groups, etc.; optionally substituted mercapto groups such as lower (C₁₋₆) alkylthio groups, C₃₋₆ cycloalkylthio groups, arylthio groups, etc.; amino groups optionally substituted with one or two the same or different substituents selected from lower (C₁₋₆) alkyl, lower (C₁₋₆) alkoxy, C₃₋₆ cycloalkyl, hydroxy, carbamoyl, phenyl, phenyl-lower (C₁₋₆) alkyl, lower (C₁₋₆) alkanoyl, C₃₋₆ cycloalkyl-carbonyl, benzoyl, phenyl-C₂₋₆ alkanoyl, lower (C₁₋₆) alkoxy-carbonyl, phenoxy-carbonyl, phenyl-C₁₋₆ alkoxy-carbonyl, lower (C₁₋₆) alkylsulfinyl, C₃₋₆ cycloalkylsulfinyl, phenylsulfinyl, lower (C₁₋₆) alkylsulfonyl, C₃₋₆ cycloalkylsulfonyl, lower (C₁₋₆) alkoxysulfonyl, and phenylsulfonyl, etc., or optionally substituted amino groups such as cycloamino groups formed by two substituents bound to each other and the nitrogen atom; imidoyl and amidino groups optionally substituted with lower (C₁₋₆) alkyl, lower (C₁₋₆) alkoxy, C₃₋₆ cycloalkyl, aryl, etc.; nitro groups; cyano groups; optionally esterified carboxyl groups such as lower alkoxycarbonyl group, aryloxycarbonyl group, aralkyloxycarbonyl group, etc.; N-mono-substituted or N,N-di-substituted carbamoyl groups optionally substituted with one or two the same or different substituents selected from lower alkyl, cycloalkyl, aryl, aralkyl, and aromatic or non-aromatic homocyclic groups and aromatic or non-aromatic heterocyclic groups, described hereinafter; optionally substituted sulfonyl groups such as lower (C₁₋₆) alkylsulfonyl, C₃₋₆ cycloalkylsulfonyl, phenyl-C₁₋₆ alkylsulfonyl, lower (C₁₋₆) alkoxysulfonyl, C₃₋₆ cycloalkyloxysulfonyl, phenyl-C₁₋₆ alkoxysulfonyl, sulfamoyl, lower (C₁₋₆) alkylaminosulfonyl, C₃₋₆ cycloalkylaminosulfonyl, phenyl-C₁₋₆ alkylaminosulfonyl, cyclicaminosulfonyl, nitroxy-C₁₋₆ alkylaminosulfonyl, anilinosulfonyl, etc.; carboxylic acid-derived acyl groups such as lower (C₁₋₆) alkyl-carbonyl (alkanoyl), C₃₋₆ cycloalkyl-carbonyl, benzoyl, etc.; sulfonic acid-derived acyl groups such as lower (C₁₋₆) alkylsulfonyl, C₃₋₆ cycloalkylsulfonyl, phenylsulfonyl, etc.; sulfinic acid-derived acyl groups such as lower (C₁₋₆) alkylsulfinyl, C₃₋₆ cycloalkylsulfinyl, phenylsulfinyl, etc.; saturated or unsaturated alicyclic hydrocarbon groups such as cycloalkyl group, cycloalkenyl group, cycloalkanedienyl group, etc.; monocyclic or fused polycyclic aromatic hydrocarbon groups such as aryl group; 5- and 6-membered aromatic monocyclic heterocyclic groups; 8- to 12-membered aromatic fused heterocyclic groups; and the like.

As the "optionally substituted mercapto group", in addition to free mercapto group, there are, for example, lower (C₁₋₆) alkylthio groups (methylthio, ethylthio, propylthio, isopropylthio, butylthio, isobutylthio, sec-butylthio, tert-butylthio, pentylthio, hexylthio and the like), C₃₋₆ cycloalkylthio groups (cyclopropylthio, cyclobutylthio, cyclopentylthio, cyclohexylthio and the like), arylthio group (phenylthio, naphthylthio and the like) and the like.

These substituted mercapto groups may further be substituted at any possible position with 1 to 3 the same or different substituents selected from, for example, halogen atoms; straight chain or branched chain aliphatic hydrocarbon groups such as alkyl group, alkenyl group, alkynyl group, etc.; optionally substituted hydroxy groups such as lower (C₁₋₆) alkoxy groups, C₃₋₆ cycloalkyloxy groups, aryloxy groups, etc.; optionally substituted mercapto groups such as lower (C₁₋₆) alkylthio groups, C₃₋₆ cycloalkylthio groups, arylthio groups, etc.; amino groups optionally substituted with one or two the same or different substituents selected from lower (C₁₋₆) alkyl, lower (C₁₋₆) alkoxy, C₃₋₆ cycloalkyl, hydroxy, carbamoyl, phenyl, phenyl-lower (C₁₋₆) alkyl, lower (C₁₋₆) alkanoyl, C₃₋₆ cycloalkyl-carbonyl, benzoyl, phenyl-C₂₋₆ alkanoyl, lower (C₁₋₆) alkoxy-carbonyl, phenoxy-carbonyl, phenyl-C₁₋₆ alkoxy-carbonyl, lower (C₁₋₆) alkylsulfinyl, C₃₋₆ cycloalkylsulfinyl, phenylsulfinyl, lower (C₁₋₆) alkylsulfonyl, C₃₋₆ cycloalkylsulfonyl, lower (C₁₋₆) alkoxysulfonyl, and phenylsulfonyl, etc., or optionally substituted amino groups such as cycloamino groups formed by two substituents bound to each other and the nitrogen atom; imidoyl and amidino groups optionally substituted with lower (C₁₋₆) alkyl, lower (C₁₋₆) alkoxy, C₃₋₆ cycloalkyl, aryl, etc.; nitro groups; cyano groups; optionally esterified carboxyl groups such as lower alkoxycarbonyl group, aryloxycarbonyl group, aralkyloxycarbonyl group, etc.; N-mono-substituted or N,N-di-substituted carbamoyl groups optionally substituted with one or two the same or different substituents selected from lower alkyl, cycloalkyl, aryl, aralkyl, and aromatic or non-aromatic homocyclic groups and aromatic or non-aromatic heterocyclic groups, described hereinafter; optionally substituted sulfonyl groups such as lower (C₁₋₆) alkylsulfonyl, C₃₋₆ cycloalkylsulfonyl, phenyl-C₁₋₆ alkylsulfonyl, lower (C₁₋₆) alkoxysulfonyl, C₃₋₆ cycloalkyloxysulfonyl, phenyl-C₁₋₆ alkoxysulfonyl, sulfamoyl, lower (C₁₋₆) alkylaminosulfonyl, C₃₋₆ cycloalkylaminosulfonyl, phenyl-C₁₋₆ alkylaminosulfonyl, cyclicaminosulfonyl, nitroxy-C₁₋₆ alkylaminosulfonyl, anilinosulfonyl, etc.; carboxylic acid-derived acyl groups such as lower (C₁₋₆) alkyl-carbonyl (alkanoyl), C₃₋₆ cycloalkyl-carbonyl, benzoyl, etc.; sulfonic acid-derived acyl groups such as lower (C₁₋₆) alkylsulfonyl, C₃₋₆ cycloalkylsulfonyl, phenylsulfonyl, etc.; sulfinic acid-derived acyl groups such as lower (C₁₋₆) alkylsulfinyl, C₃₋₆ cycloalkylsulfinyl, phenylsulfinyl, etc.; saturated or unsaturated alicyclic hydrocarbon groups such as cycloalkyl group, cycloalkenyl group, cycloalkanedienyl group, etc.; monocyclic or fused polycyclic aromatic hydrocarbon groups such as aryl group; 5- and 6-membered aromatic monocyclic heterocyclic groups; 8- to 12-membered aromatic fused heterocyclic groups; and the like.

As the "optionally substituted amino group", in addition to free amino group, there are, for example, amino group substituted with one or two the same or different substituents selected from, for example, lower (C₁₋₆) alkyl (methyl, ethyl, propyl, isopropyl, butyl, isobutyl, sec-butyl, tert-butyl, pentyl, hexyl and the like); lower (C₁₋₆) alkoxy (methoxy, ethoxy, propoxy, isopropoxy, butoxy, isobutoxy, sec-butoxy, tert-butoxy, pentyloxy, hexyloxy and the like), C₃₋₆ cycloalkyl (cyclopropyl, cyclobutyl, cyclopentyl, cyclohexyl and the like); hydroxy, carbamoyl, phenyl, phenyl-lower (C₁₋₆) alkyl (benzyl, phenethyl, 3-phenylpropyl, 4-phenylbutyl and the like); lower (C₁₋₆) alkanoyl (formyl, acetyl, propionyl, butyryl, isobutyryl, valeryl, pivaloyl and the like); C₃₋₆ cycloalkyl-carbonyl (cyclopropylcarbonyl, cyclobutylcarbonyl, cyclopentylcarbonyl, cyclohexylcarbonyl, and the like); benzoyl, phenyl-C₂₋₆ alkanoyl (phenylacetyl, phenylpropionyl and the like); lower (C₁₋₆) alkoxy-carbonyl (methoxycarbonyl, ethoxycarbonyl, propoxycarbonyl, isopropoxycarbonyl, butoxycarbonyl, isobutoxycarbonyl, tert-butoxycarbonyl, pentyloxycarbonyl, hexyloxycarbonyl, and the like); phenoxycarbonyl, phenyl-C₁₋₆ alkoxy-carbonyl (benzyloxycarbonyl, phenylethoxycarbonyl and the like); lower (C₁₋₆) alkylsulfinyl (methylsulfinyl, ethylsulfinyl, propylsulfinyl, isopropylsulfinyl, butylsulfinyl, isobutylsulfinyl, sec-butylsulfinyl, tert-butylsulfinyl, pentylsulfinyl, hexylsulfinyl, and the like); C₃₋₆ cycloalkylsulfinyl (cyclopropylsulfinyl, cyclobutylsulfinyl, cyclopentylsulfinyl, cyclohexylsulfinyl, and the like); phenylsulfinyl, lower (C₁₋₆) alkylsulfonyl (methylsulfonyl, ethylsulfonyl, propylsulfonyl, isopropylsulfonyl, butylsulfonyl, isobutylsulfonyl, tert-butylsulfonyl, sec-butylsulfonyl, pentylsulfonyl, hexylsulfonyl, and the like); C₃₋₆ cycloalkylsulfonyl (cyclopropylsulfonyl, cyclobutylsulfonyl, cyclopentylsulfonyl, cyclohexylsulfonyl, and the like); and lower (C₁₋₆) alkoxysulfonyl (methoxysulfonyl, ethoxysulfonyl, propoxysulfonyl, isopropoxysulfonyl, butoxysulfonyl, isobutoxysulfonyl, sec-butoxysulfonyl, tert-butoxysulfonyl, pentyloxysulfonyl, hexyloxysulfonyl, and the like). In some cases, two of such substituents may be bound to each other and form together with the nitrogen atom a cyclic amino group, and the cyclic amino group is, for example, pyrrolidino, piperidino, morpholino, thiomorpholino and the like.

These substituted amino groups may further be substituted at any possible position with 1 to 3 the same or different substituents selected from, for example, halogen atoms; straight chain or branched chain aliphatic hydrocarbon groups such as alkyl group, alkenyl group, alkynyl group, etc.; optionally substituted hydroxy groups such as lower (C₁₋₆) alkoxy groups, C₃₋₆ cycloalkyloxy groups, aryloxy groups, etc.; optionally substituted mercapto groups such as lower (C₁₋₆) alkylthio groups, C₃₋₆ cycloalkylthio groups, arylthio groups, etc.; amino groups optionally substituted with one or two the same or different substituents selected from lower (C₁₋₆) alkyl, lower (C₁₋₆) alkoxy, C₃₋₆ cycloalkyl, hydroxy, carbamoyl, phenyl, phenyl-lower (C₁₋₆) alkyl, lower (C₁₋₆) alkanoyl, C₃₋₆ cycloalkyl-carbonyl, benzoyl, phenyl-C₂₋₆ alkanoyl, lower (C₁₋₆) alkoxy-carbonyl, phenoxy-carbonyl, phenyl-C₁₋₆ alkoxy-carbonyl, lower (C₁₋₆) alkylsulfinyl, C₃₋₆ cycloalkylsulfinyl, phenylsulfinyl, lower (C₁₋₆) alkylsulfonyl, C₃₋₆ cycloalkylsulfonyl, lower (C₁₋₆) alkoxysulfonyl, and phenylsulfonyl, etc., or optionally substituted amino groups such as cycloamino groups formed by two substituents bound to each other and the nitrogen atom and the like; imidoyl and amidino groups optionally substituted with lower (C₁₋₆) alkyl, lower (C₁₋₆) alkoxy, C₃₋₆ cycloalkyl, aryl, etc.; nitro groups; cyano groups; optionally esterified carboxyl groups such as lower alkoxycarbonyl group, aryloxycarbonyl group, aralkyloxycarbonyl group, etc.; N-mono-substituted or N,N-di-substituted carbamoyl groups optionally substituted with one or two the same or different substituents selected from lower alkyl, cycloalkyl, aryl, aralkyl, and aromatic or non-aromatic homocyclic groups and aromatic or non-aromatic heterocyclic groups, described hereinafter; optionally substituted sulfonyl groups such as lower (C₁₋₆) alkylsulfonyl, C₃₋₆ cycloalkylsulfonyl, phenyl-C₁₋₆ alkylsulfonyl, lower (C₁₋₆) alkoxysulfonyl, C₃₋₆ cycloalkyloxysulfonyl, phenyl-C₁₋₆ alkoxysulfonyl, sulfamoyl, lower (C₁₋₆) alkylaminosulfonyl, C₃₋₆ cycloalkylaminosulfonyl, phenyl-C₁₋₆ alkylaminosulfonyl, cyclicaminosulfonyl, nitroxy-C₁₋₆ alkylaminosulfonyl, anilinosulfonyl, etc.; carboxylic acid-derived acyl groups such as lower (C₁₋₆) alkyl-carbonyl (alkanoyl), C₃₋₆ cycloalkyl-carbonyl, benzoyl, etc.; sulfonic acid-derived acyl groups such as lower (C₁₋₆) alkylsulfonyl, C₃₋₆ cycloalkylsulfonyl, phenylsulfonyl, etc.; sulfinic acid-derived acyl groups such as lower (C₁₋₆) alkylsulfinyl, C₃₋₆ cycloalkylsulfinyl, phenylsulfinyl, etc.; saturated or unsaturated alicyclic hydrocarbon groups such as cycloalkyl group, cycloalkenyl group, cycloalkanedienyl group, etc.; monocyclic or fused polycyclic aromatic hydrocarbon groups such as aryl group; 5- and 6-membered aromatic monocyclic heterocyclic groups; 8- to 12-membered aromatic fused heterocyclic groups; and the like.

Examples of the substituents of the "optionally substituted imidoyl group" and the "optionally substituted amidino group" include lower (C₁₋₆) alkyl groups (methyl, ethyl, propyl, isopropyl, butyl, isobutyl, sec-butyl, tert-butyl, pentyl, hexyl and the like); lower (C₁₋₆) alkoxy groups (methoxy, ethoxy, propoxy, isopropoxy, butoxy, isobutoxy, sec-butoxy, tert-butoxy, pentyloxy, hexyloxy and the like), C₃₋₆ cycloalkyl (cyclopropyl, cyclobutyl, cyclopentyl, cyclohexyl and the like); aryl (phenyl, naphthyl and the like), and the like.

These substituted imidoyl and substituted amidino groups may further be substituted at any possible position with 1 to 3 the same or different substituents selected from, for example, halogen atoms; straight chain or branched chain aliphatic hydrocarbon groups such as alkyl group, alkenyl group, alkynyl group and the like; optionally substituted hydroxy groups such as lower (C₁₋₆) alkoxy groups, C₃₋₆ cycloalkyloxy groups, aryloxy groups, etc.; optionally substituted mercapto groups such as lower (C₁₋₆) alkylthio groups, C₃₋₆ cycloalkylthio groups, arylthio groups, etc.; amino groups optionally substituted with one or two the same or different substituents selected from lower (C₁₋₆) alkyl, lower (C₁₋₆) alkoxy, C₃₋₆ cycloalkyl, hydroxy, carbamoyl, phenyl, phenyl-lower (C₁₋₆) alkyl, lower (C₁₋₆) alkanoyl, C₃₋₆ cycloalkyl-carbonyl, benzoyl, phenyl-C₂₋₆ alkanoyl, lower (C₁₋₆) alkoxy-carbonyl, phenoxy-carbonyl, phenyl-C₁₋₆ alkoxy-carbonyl, lower (C₁₋₆) alkylsulfinyl, C₃₋₆ cycloalkylsulfinyl, phenylsulfinyl, lower (C₁₋₆) alkylsulfonyl, C₃₋₆ cycloalkylsulfonyl, lower (C₁₋₆) alkoxysulfonyl, and phenylsulfonyl, etc., or optionally substituted amino groups such as cycloamino groups formed by two substituents bound to each other and the nitrogen atom; imidoyl and amidino groups optionally substituted with lower (C₁₋₆) alkyl, lower (C₁₋₆) alkoxy, C₃₋₆ cycloalkyl, aryl, etc.; nitro groups; cyano groups; optionally esterified carboxyl groups such as lower alkoxycarbonyl group, aryloxycarbonyl group, aralkyloxycarbonyl group, etc.; N-mono-substituted or N,N-di-substituted carbamoyl groups optionally substituted with one or two the same or different substituents selected from lower alkyl, cycloalkyl, aryl, aralkyl, and aromatic or non-aromatic homocyclic groups and aromatic or non-aromatic heterocyclic groups, described hereinafter; optionally substituted sulfonyl groups such as lower (C₁₋₆) alkylsulfonyl, C₃₋₆ cycloalkylsulfonyl, phenyl-C₁₋₆ alkylsulfonyl, lower (C₁₋₆) alkoxysulfonyl, C₃₋₆ cycloalkyloxysulfonyl, phenyl-C₁₋₆ alkoxysulfonyl, sulfamoyl, lower (C₁₋₆) alkylaminosulfonyl, C₃₋₆ cycloalkylaminosulfonyl, phenyl-C₁₋₆ alkylaminosulfonyl, cyclicaminosulfonyl, nitroxy-C₁₋₆ alkylaminosulfonyl, anilinosulfonyl, etc.; carboxylic acid-derived acyl groups such as lower (C₁₋₆) alkyl-carbonyl (alkanoyl), C₃₋₆ cycloalkyl-carbonyl, benzoyl, etc.; sulfonic acid-derived acyl groups such as lower (C₁₋₆) alkylsulfonyl, C₃₋₆ cycloalkylsulfonyl, phenylsulfonyl, etc.; sulfinic acid-derived acyl groups such as lower (C₁₋₆) alkylsulfinyl, C₃₋₆ cycloalkylsulfinyl, phenylsulfinyl, etc.; saturated or unsaturated alicyclic hydrocarbon groups such as cycloalkyl group, cycloalkenyl group, cycloalkanedienyl group, etc.; monocyclic or fused polycyclic aromatic hydrocarbon groups such as aryl group; 5- and 6-membered aromatic monocyclic heterocyclic groups; 8- to 12-membered aromatic fused heterocyclic groups; and the like.

As the "optionally esterified carboxyl groups" among the "optionally esterified or amidated carboxyl groups", in addition to free carboxyl group, there are, for example, lower alkoxycarbonyl group, aryloxycarbonyl group, aralkyloxycarbony group and the like.

Examples of the lower alkoxycarbonyl include C₁₋₆ alkoxycarbonyl groups such as methoxycarbonyl, ethoxycarbonyl, propoxycarbonyl, isopropoxycarbonyl, butoxycarbonyl, isobutoxycarbonyl, sec-butoxycarbonyl, tert-butoxycarbonyl, pentyloxycarbonyl, isopentyloxycarbonyl, neopentyloxycarbonyl, hexyloxycarbonyl, etc.; C₃₋₆ cycloalkoxycarbonyl such as cyclopropoxycarbonyl, cyclobutoxycarbonyl, cyclopentyloxycarbonyl, cyclohexyloxycarbonyl, etc.; phenyl-C₁₋₆ alkoxycarbonyl such as benzyloxycarbonyl, phenyloxycarbonyl, etc.; nitroxy-C₁₋₆ alkoxycarbonyl such as 2-nitroxyethoxycarbonyl, 3-nitroxypropoxycarbonyl, etc., and the like. Among them, C₁₋₃ alkoxycarbonyl groups such as methoxycarbonyl, ethoxycarbonyl, propoxycarbonyl, etc. are preferable.

As the aryloxycarbonyl, for example, C₇₋₁₂ aryloxycarbonyl groups such as phenoxycarbonyl, 1-naphthoxycarbonyl, 2-napthoxycarbonyl, etc. are preferable.

As the aralkyloxycarbonyl, for example, C₇₋₁₀ aralkyloxycarbonyl such as benzyloxycarbonyl, phenethyloxycarbonyl, etc. (e.g., C₆₋₁₀ aryl-C₁₋₄ alkoxycarbonyl) are preferable.

These esterified carboxyl groups may further be substituted at any possible position with 1 to 3 the same or different substituents selected from, for example, halogen atoms; straight chain or branched chain aliphatic hydrocarbon groups such as alkyl group, alkenyl group, alkynyl group, etc.; optionally substituted hydroxy groups such as lower (C₁₋₆) alkoxy groups, C₃₋₆ cycloalkyloxy groups, aryloxy groups, etc.; optionally substituted mercapto groups such as lower (C₁₋₆) alkylthio groups, C₃₋₆ cycloalkylthio groups, arylthio groups, etc.; amino groups optionally substituted with one or two the same or different substituents selected from lower (C₁₋₆) alkyl, lower (C₁₋₆) alkoxy, C₃₋₆ cycloalkyl, hydroxy, carbamoyl, phenyl, phenyl-lower (C₁₋₆) alkyl, lower (C₁₋₆) alkanoyl, C₃₋₆ cycloalkyl-carbonyl, benzoyl, phenyl-C₂₋₆ alkanoyl, lower (C₁₋₆) alkoxy-carbonyl, phenoxy-carbonyl, phenyl-C₁₋₆ alkoxy-carbonyl, lower (C₁₋₆) alkylsulfinyl, C₃₋₆ cycloalkylsulfinyl, phenylsulfinyl, lower (C₁₋₆) alkylsulfonyl, C₃₋₆ cycloalkylsulfonyl, lower (C₁₋₆) alkoxysulfonyl, and phenylsulfonyl, etc., or optionally substituted amino groups such as cycloamino groups formed by two substituents bound to each other and the nitrogen atom; imidoyl and amidino groups optionally substituted with lower (C₁₋₆) alkyl, lower (C₁₋₆) alkoxy, C₃₋₆ cycloalkyl, aryl, etc.; nitro groups; cyano groups; optionally esterified carboxyl groups such as lower alkoxycarbonyl group, aryloxycarbonyl group, aralkyloxycarbonyl group, etc.; N-mono-substituted or N,N-di-substituted carbamoyl groups optionally substituted with one or two the same or different substituents selected from lower alkyl, cycloalkyl, aryl, aralkyl, and aromatic or non-aromatic homocyclic groups and aromatic or non-aromatic heterocyclic groups, described hereinafter; optionally substituted sulfonyl groups such as lower (C₁₋₆) alkylsulfonyl, C₃₋₆ cycloalkylsulfonyl, phenyl-C₁₋₆ alkylsulfonyl, lower (C₁₋₆) alkoxysulfonyl, C₃₋₆ cycloalkyloxysulfonyl, phenyl-C₁₋₆ alkoxysulfonyl, sulfamoyl, lower (C₁₋₆) alkylaminosulfonyl, C₃₋₆ cycloalkylaminosulfonyl, phenyl-C₁₋₆ alkylaminosulfonyl, cyclicaminosulfonyl, nitroxy-C₁₋₆ alkylaminosulfonyl, anilinosulfonyl, etc.; carboxylic acid-derived acyl groups such as lower (C₁₋₆) alkyl-carbonyl (alkanoyl), C₃₋₆ cycloalkyl-carbonyl, benzoyl, etc.; sulfonic acid-derived acyl groups such as lower (C₁₋₆) alkylsulfonyl, C₃₋₆ cycloalkylsulfonyl, phenylsulfonyl, etc.; sulfinic acid-derived acyl groups such as lower (C₁₋₆) alkylsulfinyl, C₃₋₆ cycloalkylsulfinyl, phenylsulfinyl, etc.; saturated or unsaturated alicyclic hydrocarbon groups such as cycloalkyl group, cycloalkenyl group, cycloalkanedienyl group, etc.; monocyclic or fused polycyclic aromatic hydrocarbon groups such as aryl group; 5- and 6-membered aromatic monocyclic heterocyclic groups; 8- to 12-membered aromatic fused heterocyclic groups; and the like.

As the "amidated carboxyl group", in addition to carbamoyl group, there are, for example, N-monosubstituted carbamoyl groups and N,N-di-substituted carbamoyl groups.

The "N-monosubstituted carbamoyl group" means carbamoyl group having one substituent at the nitrogen atom and examples of the substituent include lower alkyl groups (e.g., C₁₋₆ alkyl such as methyl, ethyl, propyl, isopropyl, butyl, isobutyl, tert-butyl, pentyl, hexyl and the like); cycloalkyl groups (e.g., C₃₋₆ cycloalkyl such as cyclopropyl, cyclobutyl, cyclopentyl, cyclohexyl); aryl groups (e.g., C₆₋₁₀ aryl such as phenyl, 1-naphthyl, 2-naphthyl); aralkyl groups (e.g. C₇₋₁₀ aralkyl such as benzyl, phenethyl, preferably phenyl-C₁₋₄ alkyl); and heterocyclic groups described hereinafter, and the like.

Preferred N-mono-lower (C₁₋₆) alkylcarbamoyl includes, for example, methylcarbamoyl, ethylcarbamoyl, propylcarbamoyl, isopropylcarbamoyl, butylcarbamoyl, isobutylcarbamoyl, sec-butylcarbamoyl, tert-butylcarbamoyl, pentylcarbamoyl, hexylcarbamoyl, and the like.

The N,N-disubstituted carbamoyl means carbamoyl having two substituent groups at nitrogen atom, and examples of one the substituent groups are the same as the substituent of the above-mentioned "N-monosubstituted carbamoyl", and examples of the other include lower alkyl groups (e.g., C₁₋₆ alkyl such as methyl, ethyl, propyl, isopropyl, butyl, isobutyl, t-butyl, pentyl, hexyl, etc.); C₃₋₆ cycloalkyl groups (e.g., cyclopropyl, cyclobutyl, cyclopentyl, cyclohexyl, etc.), C₇₋₁₀ aralkyl groups (e.g., benzyl, phenethyl, etc., preferably phenyl-C₁₋₄ alkyl, etc.) and the like. Also, the two substituents may form a cyclic amino group together with the nitrogen atom in some cases, and in such cases, examples of the cyclic aminocarbamoyl group include 3- to 8-membered (preferably 5- and 6-membered) cyclic aminocarbonyl groups such as 1-azetidiylcarbonyl, 1-pyrrolidinylcarbonyl, piperidinocarbonyl, morpholinocarbonyl, 1-piperadinylcarbonyl, 1-piperadinylcarbonyl which may further be substituted at 4-position with a lower alkyl group (e.g., C₁₋₆ alkyl group such as methyl, ethyl, propyl, isopropyl, butyl, t-butyl, pentyl, hexyl, etc.); aralkyl group (e.g., C₇₋₁₀ aralkyl group such as benzyl, phenethyl, etc.); aryl group (e.g., C₆₋₁₀ aryl group such as phenyl, 1-naphthyl, 2-naphthyl, etc.); and the like. Preferred N,N-di-lower (C₁₋₆) alkylcarbamoyl are, for example, N,N-dimethylcarbamoyl, N,N-diethylcarbamoyl, N,N-dipropylcarbamoyl, N,N-dibutylcarbamoyl, and the like, and preferred C₃₋₆ cycloalkylcarbamoyl are, for example, cyclopropylcarbamoyl, cyclobutylcarbamoyl, cyclopentylcarbamoyl, cyclohexylcarbamoyl and the like, and preferred phenyl-C₁₋₆ alkylcarbamoyl are, for example, benzylcarbamoyl, phenethylcarbamoyl and the like, and preferred nitroxyC₁₋₆ alkylaminocarbonyl are, for example, 2-nitroxyethylcarbamoyl, 3-nitroxypropylcarbamoyl and the like, and preferred cyclic aminocarbonyl are, for example, morpholinocarbonyl, piperidinocarbonyl, pyridinocarbonyl, thiomorpholinocarbonyl and the like. Further, anilinocarbonyl, etc. can also be exemplified.

These amidated carboxyl groups may further be substituted at any possible position with 1 to 3 the same or different substituents selected from, for example, halogen atoms; straight chain or branched chain aliphatic hydrocarbon groups such as alkyl group, alkenyl group, alkynyl group, etc.; optionally substituted hydroxy groups such as lower (C₁₋₆) alkoxy groups, C₃₋₆ cycloalkyloxy groups, aryloxy groups, etc.; optionally substituted mercapto groups such as lower (C₁₋₆) alkylthio groups, C₃₋₆ cycloalkylthio groups, arylthio groups, etc.; amino groups optionally substituted with one or two the same or different substituents selected from lower (C₁₋₆) alkyl, lower (C₁₋₆) alkoxy, C₃₋₆ cycloalkyl, hydroxy, carbamoyl, phenyl, phenyl-lower (C₁₋₆) alkyl, lower (C₁₋₆) alkanoyl, C₃₋₆ cycloalkyl-carbonyl, benzoyl, phenyl-C₂₋₆ alkanoyl, lower (C₁₋₆) alkoxy-carbonyl, phenoxy-carbonyl, phenyl-C₁₋₆ alkoxy-carbonyl, lower (C₁₋₆) alkylsulfinyl, C₃₋₆ cycloalkylsulfinyl, phenylsulfinyl, lower (C₁₋₆) alkylsulfonyl, C₃₋₆ cycloalkylsulfonyl, lower (C₁₋₆) alkoxysulfonyl, and phenylsulfonyl, etc., or optionally substituted amino groups such as cycloamino groups formed by two substituents bound to each other and the nitrogen atom; imidoyl and amidino groups optionally substituted with lower (C₁₋₆) alkyl, lower (C₁₋₆) alkoxy, C₃₋₆ cycloalkyl, aryl, etc.; nitro groups; cyano groups; optionally esterified carboxyl groups such as lower alkoxycarbonyl group, aryloxycarbonyl group, aralkyloxycarbonyl group, etc.; N-mono-substituted or N,N-di-substituted carbamoyl groups optionally substituted with one or two the same or different substituents selected from lower alkyl, cycloalkyl, aryl, aralkyl, and aromatic or non-aromatic homocyclic groups and aromatic or non-aromatic heterocyclic groups, described hereinafter; optionally substituted sulfonyl groups such as lower (C₁₋₆) alkylsulfonyl, C₃₋₆ cycloalkylsulfonyl, phenyl-C₁₋₆ alkylsulfonyl, lower (C₁₋₆) alkoxysulfonyl, C₃₋₆ cycloalkyloxysulfonyl, phenyl-C₁₋₆ alkoxysulfonyl, sulfamoyl, lower (C₁₋₆) alkylaminosulfonyl, C₃₋₆ cycloalkylaminosulfonyl, phenyl-C₁₋₆ alkylaminosulfonyl, cyclicaminosulfonyl, nitroxy-C₁₋₆ alkylaminosulfonyl, anilinosulfonyl, etc.; carboxylic acid-derived acyl groups such as lower (C₁₋₆) alkyl-carbonyl (alkanoyl), C₃₋₆ cycloalkyl-carbonyl, benzoyl, etc.; sulfonic acid-derived acyl groups such as lower (C₁₋₆) alkylsulfonyl, C₃₋₆ cycloalkylsulfonyl, phenylsulfonyl, etc.; sulfinic acid-derived acyl groups such as lower (C₁₋₆) alkylsulfinyl, C₃₋₆ cycloalkylsulfinyl, phenylsulfinyl, etc.; saturated or unsaturated alicyclic hydrocarbon groups such as cycloalkyl group, cycloalkenyl group, cycloalkanedienyl group, etc.; monocyclic or fused polycyclic aromatic hydrocarbon groups such as aryl group; 5- and 6-membered aromatic monocyclic heterocyclic groups; 8- to 12-membered aromatic fused heterocyclic groups; and the like.

Examples of the "optionally substituted sulfonyl" include lower (C₁₋₆) alkylsulfonyl (e.g., methylsulfonyl, ethylsulfonyl, propylsulfonyl, isopropylsulfonyl, butylsulfonyl, isobutylsulfonyl, sec-butylsulfonyl, tert-butylsulfonyl, pentylsulfonyl, hexylsulfonyl and the like); C₃₋₆ cycloalkylsulfonyl (e.g., cyclopropylsulfonyl, cyclobutylsulfonyl, cyclopentylsulfonyl, cyclohexylsulfonyl and the like); phenyl-C₁₋₆ alkylsulfonyl (e.g., benzylsulfonyl, phenethylsulfonyl and the like); lower (C₁₋₆) alkoxysulfonyl (e.g., methoxysulfonyl, ethoxysulfonyl, propoxysulfonyl, isopropoxysulfonyl, butoxysulfonyl, isobutoxysulfonyl, sec-butoxysulfonyl, tert-butoxysulfonyl, pentyloxysulfonyl, hexyloxysulfonyl and the like); C₃₋₆ cycloalkyloxysulfonyl (e.g., cyclopropoxysulfonyl, cyclobutyloxysulfonyl, cyclopentyloxysulfonyl, cyclohexyloxysulfonyl and the like); phenyl-C₁₋₆ alkoxysulfonyl (e.g., benzyloxysulfonyl, phenethyloxysulfonyl and the like); sulfamoyl, lower (C₁₋₆) alkylaminosulfonyl (e.g., methylaminosulfonyl, ethylaminosulfonyl, propylaminosulfonyl, isopropylaminosulfonyl, butylaminosulfonyl, isobutylaminosulfonyl, sec-butylaminosulfonyl, tert-butylaminosulfonyl, pentylaminosulfonyl, hexylaminosulfonyl and the like); C₃₋₆ cycloalkylaminosulfonyl (e.g., cyclopropylaminosulfonyl, cyclobutylaminosulfonyl, cyclopentylaminosulfonyl, cyclohexylylaminosulfonyl and the like); phenyl-C₁₋₆ alkylaminosulfonyl (e.g., benzylaminosulfonyl, phenethylaminosulfonyl and the like); cyclicaminosulfonyl (e.g., morpholinosulfonyl, piperidinosulfonyl, pyrrolidinosulfonyl, thiomorpholinosulfonyl and the like); nitroxyC₁₋₆ alkylaminosulfonyl (e.g., 2-nitroxyethylaminosulfonyl, 3-nitroxypropylaminosulfonyl, and the like); anilinosulfonyl; and the like.

Examples of the "acyl groups derived from a carboxylic acid" include lower (C₁₋₆) alkyl-carbonyl (alkanoyl) (e.g., formyl, acetyl, propionyl, butyryl, isobutyryl, valeryl, pivaloyl and the like); C₃₋₆ cycloalkyl-carbonyl (e.g., cyclopropylcarbonyl, cyclobutylcarbonyl, cyclopentylcarbonyl, cyclohexylcarbonyl, and the like); benzoyl, and the like.

Examples of the "acyl groups derived from a sulfonic acid" include lower (C₁₋₆) alkylsulfonyl (e.g., methysulfonyl, ethylsulfonyl, propylsulfonyl, isopropylsulfonyl, butylsulfonyl, isobutylsulfonyl, sec-butylsulfonyl, tert-butylsulfonyl, pentylsulfonyl, hexylsulfonyl, and the like); C₃₋₆ cycloalkylsulfonyl (e.g. cyclopropylsulfonyl, cyclobutylsulfonyl, cyclopentylsulfonyl, cyclohexylsulfonyl, and the like); phenylsulfonyl; and the like.

Examples of the "acyl groups derived from a sulfinic acid" include lower (C₁₋₆) alkylsulfinyl (e.g., methysulfinyl, ethylsulfinyl, propylsulfinyl, isopropylsulfinyl, butylsulfinyl, isobutylsulfinyl, sec-butylsulfinyl, tert-butylsulfinyl, pentylsulfinyl, hexylsulfinyl, and the like); C₃₋₆ cycloalkylsulfinyl (e.g. cyclopropylsulfinyl, cyclobutylsulfinyl, cyclopentylsulfinyl, cyclohexylsulfinyl, and the like); phenylsulfinyl; and the like.

As mentioned above, the exemplified respective "acyl groups" may further be substituted at any possible position with 1 to 3 the same or different substituents selected from, for example, halogen atoms; straight chain or branched chain aliphatic hydrocarbon groups such as alkyl group, alkenyl group, alkynyl group, etc.; optionally substituted hydroxy groups such as lower (C₁₋₆) alkoxy groups, C₃₋₆ cycloalkyloxy groups, aryloxy groups, etc.; optionally substituted mercapto groups such as lower (C₁₋₆) alkylthio groups, C₃₋₆ cycloalkylthio groups, arylthio groups, etc.; amino groups optionally substituted with one or two the same or different substituents selected from lower (C₁₋₆) alkyl, lower (C₁₋₆) alkoxy, C₃₋₆ cycloalkyl, hydroxy, carbamoyl, phenyl, phenyl-lower (C₁₋₆) alkyl, lower (C₁₋₆) alkanoyl, C₃₋₆ cycloalkyl-carbonyl, benzoyl, phenyl-C₂₋₆ alkanoyl, lower (C₁₋₆) alkoxy-carbonyl, phenoxy-carbonyl, phenyl-C₁₋₆ alkoxy-carbonyl, lower (C₁₋₆) alkylsulfinyl, C₃₋₆ cycloalkylsulfinyl, phenylsulfinyl, lower (C₁₋₆) alkylsulfonyl, C₃₋₆ cycloalkylsulfonyl, lower (C₁₋₆) alkoxysulfonyl, and phenylsulfonyl, etc., or optionally substituted amino groups such as cycloamino groups formed by two substituents bound to each other and the nitrogen atom; imidoyl and amidino groups optionally substituted with lower (C₁₋₆) alkyl, lower (C₁₋₆) alkoxy, C₃₋₆ cycloalkyl, aryl, etc.; nitro groups; cyano groups; optionally esterified carboxyl groups such as lower alkoxycarbonyl group, aryloxycarbonyl group, aralkyloxycarbonyl group, etc.; N-mono-substituted or N,N-di-substituted carbamoyl groups optionally substituted with one or two the same or different substituents selected from lower alkyl, cycloalkyl, aryl, aralkyl, and aromatic or non-aromatic homocyclic groups and aromatic or non-aromatic heterocyclic groups, described hereinafter; optionally substituted sulfonyl groups such as lower (C₁₋₆) alkylsulfonyl, C₃₋₆ cycloalkylsulfonyl, phenyl-C₁₋₆ alkylsulfonyl, lower (C₁₋₆) alkoxysulfonyl, C₃₋₆ cycloalkyloxysulfonyl, phenyl-C₁₋₆ alkoxysulfonyl, sulfamoyl, lower (C₁₋₆) alkylaminosulfonyl, C₃₋₆ cycloalkylaminosulfonyl, phenyl-C₁₋₆ alkylaminosulfonyl, cyclicaminosulfonyl, nitroxy-C₁₋₆ alkylaminosulfonyl, anilinosulfonyl, etc.; carboxylic acid-derived acyl groups such as lower (C₁₋₆) alkyl-carbonyl (alkanoyl), C₃₋₆ cycloalkyl-carbonyl, benzoyl, etc.; sulfonic acid-derived acyl groups such as lower (C₁₋₆) alkylsulfonyl, C₃₋₆ cycloalkylsulfonyl, phenylsulfonyl, etc.; sulfinic acid-derived acyl groups such as lower (C₁₋₆) alkylsulfinyl, C₃₋₆ cycloalkylsulfinyl, phenylsulfinyl, etc.; saturated or unsaturated alicyclic hydrocarbon groups such as cycloalkyl group, cycloalkenyl group, cycloalkanedienyl group, etc.; monocyclic or fused polycyclic aromatic hydrocarbon groups such as aryl group; 5- and 6-membered aromatic monocyclic heterocyclic groups; 8- to 12-membered aromatic fused heterocyclic groups; and the like.

Examples of the "aromatic or non-aromatic homocyclic group" include saturated or unsaturated aliphatic hydrocarbon group such as cycloalkyl group, cycloalkenyl group, cycloalkanedienyl group, etc., and aryl group such as monocyclic or fused polycyclic aromatic hydrocarbon group, etc.; and examples of cycloalkyl group include C₃₋₉ cycloalkyl group such as cyclopropyl, cyclobutyl, cyclopentyl, cyclohexyl, cycloheptyl, cyclooctyl, cyclononyl, etc. Examples of cycloalkenyl group include C₃₋₆ cycloalkenyl group such as 2-cyclopenten-1-yl, 3-cyclopenten-1-yl, 2-cyclohexen-1-yl, 3-cyclohexen-1-yl, 1-cyclobuten-1-yl, 1-cyclopenten-1-yl, etc. Examples of cycloalkanedienyl include C₄₋₆ cycloalkanedienyl group such as 2,4-cyclopentanedien-1-yl, 2,4-cyclohexanedien-1-yl, 2,5-cyclohexanedien-1-yl, etc. Examples of aryl group include C₆₋₁₄ aryl group such as phenyl, 1-naphthyl, 2-naphthyl, anthryl, phenanthryl, acenaphthylenyl, etc. Among them, optionally substituted 5- or 6-membered aromatic or non-aromatic homocyclic groups are preferred and optionally substituted 5- or 6-membered aromatic homocyclic groups are more preferred.

Examples of an aromatic heterocyclic ring of the "aromatic or non-aromatic heterocyclic group" include an aromatic heterocyclic ring containing at least one (preferably 1 to 3, more preferably 1 or 2) hetero atom selected from 1 to 3 kinds (preferably one kind to two kinds) of atoms selected from oxygen, sulfur, and nitrogen atoms as the atom which forms the ring system (ring atom), etc. Specific examples thereof include aromatic monocyclic heterocyclic group (e.g., 5- or 6-membered aromatic monocyclic heterocyclic group such as furyl, thienyl, pyrrolyl, oxazolyl, isoxazolyl, thiazolyl isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyridazinyl, pyrimidinyl, pyrazinyl, triazinyl and the like); aromatic fused heterocyclic group (e.g., 8- to 12-membered aromatic fused heterocyclic group such as benzofuranyl, isobenzofuranyl, benzothienyl, indolyl, isoindolyl, 1H-indazolyl, benzindazolyl, benzoxazolyl, 1,2-benzoisooxazolyl, benzothiazolyl, benzopyranyl, 1,2-benzoisothiazolyl, 1H-benzotriazolyl, quinolyl, isoquinolyl, cinnolinyl, quinazolinyl, quinoxalinyl, phthalazinyl, naphthyldinyl, purinyl, buteridinyl, carbazolyl, α-carbolinyl, β-carbolinyl, γ-carbolinyl, acridinyl, phenoxazinyl, phenothiazinyl, phenazinyl, phenoxathiinyl, thianthrenyl, phenathridinyl, phenathrolinyl, indolizinyl, pyrrolo[1,2-b]pyridazinyl, pyrazolo[1,5-a]pyridyl, imidazo[1,2-a]pyridyl, imodazo[1,5-a]pyridyl, imidazo[1,2-b]pyridazinyl, imidazo[1,2-a]pyrimidinyl, 1,2,4-triazolo[4,3-a]pyridyl, 1,2,4-triazolo[4,3-b]pyridazinyl, and the like); and the like. Among them, optionally substituted 5- or 6-membered aromatic or non-aromatic heterocyclic groups are preferred and optionally substituted 5- or 6-membered aromatic heterocyclic groups are more preferred.

Examples of the "non-aromatic heterocyclic group" include saturated or unsaturated non-aromatic heterocyclic groups (aliphatic heterocyclic group) containing at least one (preferably 1 to 4, more preferably 1 or 2) heteroatom selected from one to three kinds (preferably one to two kinds) of atoms selected from oxygen, sulfur, and nitrogen atoms as the atom (ring atom) forming the ring, and examples are 3- to 8-membered (preferably 5- or 6-membered) saturated or unsaturated (preferably saturated) non-aromatic heterocyclic rings (aliphatic heterocyclic rings) such as oxiranyl, azetidinyl, oxetanyl, thiethanyl, pyrrolidinyl, tetrahydrofuryl, thiolanyl, piperidyl, tetrahydropyranyl, morpholinyl, thiomorpholinyl, piperazinyl.

In the above-mentioned heterocyclic group, nitrogen atom constituting the ring may be oxidized and if the ring is a nitrogen-containing aromatic heterocyclic ring having hydroxy group as its substituent such as 2-oxypyridine, the ring may be in the form of a nitrogen-containing aromatic heterocyclic ring having oxo group such as α-pyridone (equivalent to a nitrogen-containing aromatic heterocyclic group having hydroxy group as its substituent in terms of the chemical structure), and in the case, the ring is a nitrogen-containing aromatic heterocyclic ring having oxo group, the above-mentioned substituent may exist on the nitrogen atom of the ring.

These aromatic or non-aromatic homocyclic groups and aromatic or non-aromatic heterocyclic groups may further be substituted at any possible position with 1 to 3 the same or different substituents selected from, for example, halogen atoms; straight chain or branched chain aliphatic hydrocarbon groups such as alkyl group, alkenyl group, alkynyl group, etc.; optionally substituted hydroxy groups such as lower (C₁₋₆) alkoxy groups, C₃₋₆ cycloalkyloxy groups, aryloxy groups, etc.; optionally substituted mercapto groups such as lower (C₁₋₆) alkylthio groups, C₃₋₆ cycloalkylthio groups, arylthio groups, etc.; amino groups optionally substituted with one or two the same or different substituents selected from lower (C₁₋₆) alkyl, lower (C₁₋₆) alkoxy, C₃₋₆ cycloalkyl, hydroxy, carbamoyl, phenyl, phenyl-lower (C₁₋₆) alkyl, lower (C₁₋₆) alkanoyl, C₃₋₆ cycloalkyl-carbonyl, benzoyl, phenyl-C₂₋₆ alkanoyl, lower (C₁₋₆) alkoxy-carbonyl, phenoxy-carbonyl, phenyl-C₁₋₆ alkoxy-carbonyl, lower (C₁₋₆) alkylsulfinyl, C₃₋₆ cycloalkylsulfinyl, phenylsulfinyl, lower (C₁₋₆) alkylsulfonyl, C₃₋₆ cycloalkylsulfonyl, lower (C₁₋₆) alkoxysulfonyl, and phenylsulfonyl, etc., or optionally substituted amino groups such as cycloamino groups formed by two substituents bound to each other and the nitrogen atom; imidoyl and amidino groups optionally substituted with lower (C₁₋₆) alkyl, lower (C₁₋₆) alkoxy, C₃₋₆ cycloalkyl, aryl, etc.; nitro groups; cyano groups; optionally esterified carboxyl groups such as lower alkoxycarbonyl group, aryloxycarbonyl group, aralkyloxycarbonyl group, etc.; N-mono-substituted or N,N-di-substituted carbamoyl groups optionally substituted with one or two the same or different substituents selected from lower alkyl, cycloalkyl, aryl, aralkyl, and aromatic or non-aromatic homocyclic groups and aromatic or non-aromatic heterocyclic groups, described hereinafter; optionally substituted sulfonyl groups such as lower (C₁₋₆) alkylsulfonyl, C₃₋₆ cycloalkylsulfonyl, phenyl-C₁₋₆ alkylsulfonyl, lower (C₁₋₆) alkoxysulfonyl, C₃₋₆ cycloalkyloxysulfonyl, phenyl-C₁₋₆ alkoxysulfonyl, sulfamoyl, lower (C₁₋₆) alkylaminosulfonyl, C₃₋₆ cycloalkylaminosulfonyl, phenyl-C₁₋₆ alkylaminosulfonyl, cyclicaminosulfonyl, nitroxy-C₁₋₆ alkylaminosulfonyl, anilinosulfonyl, etc.; carboxylic acid-derived acyl groups such as lower (C₁₋₆) alkyl-carbonyl (alkanoyl), C₃₋₆ cycloalkyl-carbonyl, benzoyl, etc.; sulfonic acid-derived acyl groups such as lower (C₁₋₆) alkylsulfonyl, C₃₋₆ cycloalkylsulfonyl, phenylsulfonyl, etc.; sulfinic acid-derived acyl groups such as lower (C₁₋₆) alkylsulfinyl, C₃₋₆ cycloalkylsulfinyl, phenylsulfinyl, etc.; saturated or unsaturated alicyclic hydrocarbon groups such as cycloalkyl group, cycloalkenyl group, cycloalkanedienyl group, etc.; monocyclic or fused polycyclic aromatic hydrocarbon groups such as aryl group; 5- and 6-membered aromatic monocyclic heterocyclic groups; 8- to 12-membered aromatic fused heterocyclic groups; and the like.

In the cyclic 1,3-diketone derivative having a symmetry plane to be used for the production process of the present invention, more specifically, the compound represented by the formula (I), R¹ to R⁴ and R⁷ and R⁸ can be selected widely from the above-described substituents, and preferably a halogen atom, an optionally substituted straight chain or branched chain aliphatic hydrocarbon group, an optionally substituted hydroxy, an optionally substituted mercapto group, an optionally substituted amino group, an optionally substituted imidoyl group, an optionally substituted amidino group, nitro group, cyano group, an optionally esterified or amidated carboxyl group, an optionally substituted sulfonyl group, an acyl group derived from a carboxylic acid, an acyl group derived from a sulfonic acid, an acyl groups derived from a sulfinic acid, an optionally substituted aromatic or non-aromatic homocyclic group, and an optionally substituted aromatic or non-aromatic heterocyclic group; more preferably a halogen atom, an optionally substituted straight chain or branched chain aliphatic hydrocarbon group, an optionally substituted hydroxy group, an optionally substituted aromatic or non-aromatic homocyclic group, and an optionally substituted aromatic or non-aromatic heterocyclic group; and especially preferably a lower (C₁₋₆) alkyl and an optionally substituted 5- or 6-membered or aromatic homocyclic or heterocyclic group; specifically preferred examples are optionally substituted methyl, ethyl, propyl, phenyl, pyrrolyl, furyl, thienyl and the like.

Further, R⁰, R⁵, R⁶, R⁹ and R¹⁰ can be selected widely from the above-mentioned substituent groups, and preferably , it is a halogen atom, an optionally substituted straight chain or branched chain aliphatic hydrocarbon group, an optionally substituted hydroxy group, and an optionally substituted mercapto group; and more preferably, it is hydrogen atom.

The examples of the cyclic 1,3-diketone derivative having a symmetry plane to be especially preferably used for the production process of the present invention are as follows.
5-phenylcyclohexane-1,3-dione;
5-(2-fluorophenyl)cyclohexane-1,3-dione;
5-(4-fluorophenyl)cyclohexane-1,3-dione;
5-(2,4-difluorophenyl)cyclohexane-1,3-dione;
5-(2-chlorophenyl)cyclohexane-1,3-dione;
5-(2,3-dichlorophenyl)cyclohexane-1,3-dione;
5-(2,6-dichlorophenyl)cyclohexane-1,3-dione;
5-(2-bromophenyl)cyclohexane-1,3-dione;
5-(3-bromophenyl)cyclohexane-1,3-dione;
5-(2-methylphenyl)cyclohexane-1,3-dione;
5-(2-trifluoromethylphenyl)cyclohexane-1,3-dione;
5-(2,5-dimethylphenyl)cyclohexane-1,3-dione;
2-(1-hydroxyethylidene)-5-(2-chlorophenyl)cyclohexane-1,3-dione;
5-(2-methoxyphenyl)cyclohexane-1,3-dione;
5-(4-methoxyphenyl)cyclohexane-1,3-dione;
5-(2-furyl)cyclohexane-1,3-dione;
5-(2-thienyl)cyclohexane-1,3-dione;
5-(3-thienyl)cyclohexane-1,3-dione;
5-(3-methyl-2-thienyl)cyclohexane-1,3-dione;
5-(5-methyl-2-thienyl)cyclohexane-1,3-dione;
5-(3-chloro-2-thienyl)cyclohexane-1,3-dione;
5-(5-chloro-2-thienyl)cyclohexane-1,3-dione;
5-(2-pyridyl)cyclohexane-1,3-dione;
5-(4-pyridyl)cyclohexane-1,3-dione;
5-(5-fluoro-2-methylphenyl)cyclohexane-1,3-dione; and the like.

Further, the following compounds can also be preferably used for the production process of the present invention.
5-(2-chlorophenyl)-2-propionylcyclohexane-1,3-dione;
2-(1-hydroxyethylidene)-5-(2-methylphenyl)cyclohexane-1,3-dione;
2-acetyl-5-(2-methoxyphenyl)cyclohexane-1,3-dione;
2-acetyl-5-(2-chlorophenyl)cyclohexane-1,3-dione;
4,5-dimethylcyclopentane-1,3-dione;
5,6-diphenylcycloheptane-1,3-dione; and the like.

In addition, the cyclic 1,3-diketone derivative having a symmetry plane, and the chiral cyclic enaminone derivative to be used for the production process of the present invention respectively have tautomers, and all of these tautomers are included in the scope of the present invention.

The production process of the present invention can schematically be illustrated as following formulas: 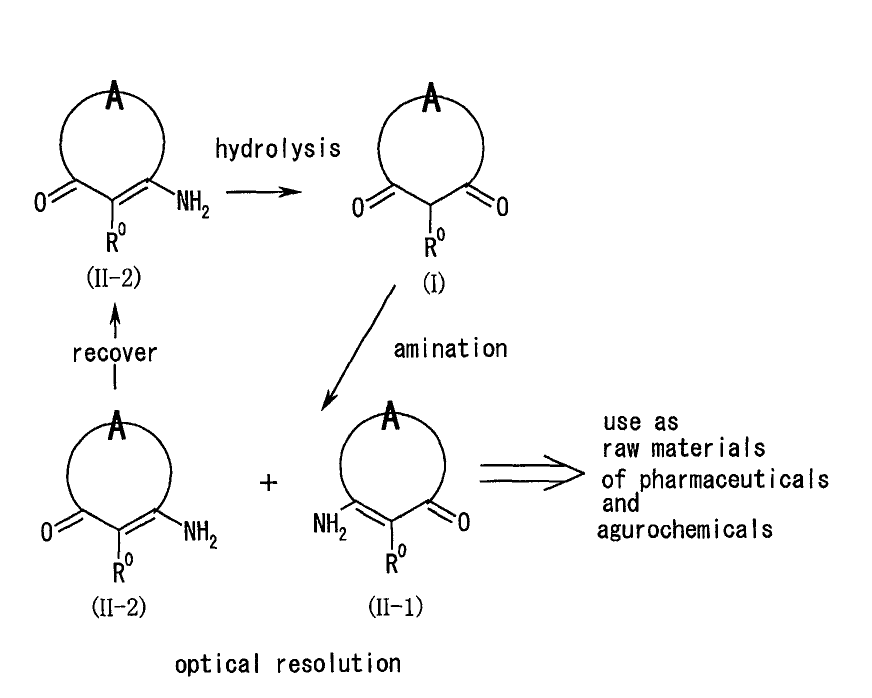 wherein the partial structure: 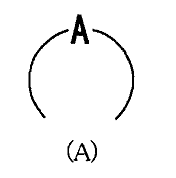 of the respective compounds of the reaction formulas is as defined above.

That is, an optical isomeric mixture of chiral and cyclic enaminone derivatives represented by the formulas (II-1) and (II-2) is obtained by aminating one carbonyl of the diketone of the cyclic 1,3-diketone derivative having a symmetry plane of the present invention represented by the formula (I). Then, the optically active isomer, for example, represented by the formula (II-1), obtained by optically resolving the aiming optically active cyclic enaminone derivative from the above-mentioned optical isomeric mixture is used as a synthetic raw material to produce the desired pharmaceutical product. On the other hand, the remaining optically active isomer represented by the formula (II-2) can be recovered and subjected to hydrolysis reaction to obtain the cyclic 1,3-diketone derivative having a symmetry plane represented by the formula (I), which is originally used as the raw material for the present invention. Thus obtained compound represented by the formula (I) can be again subjected to the above-mentioned amination reaction to finally convert the compound represented by the formula (I) entirely into the desired optically active cyclic enaminone derivative represented by the formula (II-1).

The amination of the present invention can be carried out in a manner similar to those described in a journal, Synthesis, vol. 11, p.902, (1983). Specifically, it is carried out by stirring a cyclic 1,3-diketone derivative having a symmetry plane, e.g., the compound represented by the formula (I) and an ammonium salt (e.g., ammonium salts selected from amination agents such as ammonium formate, ammonium acetate, ammonium chloride, ammonium sulfate) in about 1 to 30 equivalent, preferably about 1 to 10 equivalent in an inert solvent, for example, methanol, ethanol, benzene, toluene, chloroform, dichloromethane, 1,2-dichloroethane, tetrahydrofuran, diethylether, hexane, ethyl acetate, dimethylformamide and the like, or their mixture, preferably an alcohol type solvent such as ethanol at about 0°C to refluxing temperature of the solvent, preferably from a room temperature to about 100°C, for 0.5 to 48 hours.

By the above-mentioned amination reaction, an optical isomeric mixture of the chiral and cyclic enaminone derivative, for example, a mixture of the optical isomers represented by the formulas (II-1) and (II-2), can be obtained.

In addition, when the cyclic 1,3-diketone derivative having a symmetry plane, for example, the compound represented by the formula (I) has a substituent liable to be affected with the above-mentioned amination reaction, the substituent can be protected by a known method if necessary.

Then, the obtained optical isomeric mixture of the chiral and cyclic enaminone derivative can be subjected to an optical resolution method to obtain the desired optically active cyclic enaminone derivative. That is, the optical isomeric mixture of the cyclic enaminone derivative is reacted with an optical resolution agent, preferably an optical resolution agent with a high acidity to resolve in the form of a salt with the optical resolution agent. As the optical resolution agent with a high acidity to be used herein, there can be used that having a dissociation constant pKa in water of about 4 or lower, preferably about -10 to 3. More specifically, for example, an optically active phosphoric acid derivative, an optically active sulfamic acid derivative, and an optically active sulfonic acid derivative are preferred.

Examples of the optically active phosphoric acid derivative include optically active isomers of a compound of the formula: 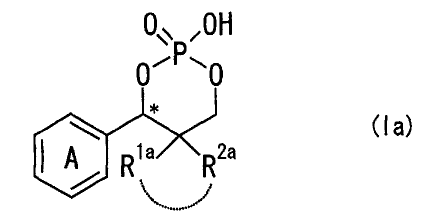 wherein A ring denotes a optionally substituted benzene ring; R^{1a} and R^{2a} independently denote hydrogen atom, a halogen atom, an optionally substituted hydrocarbon group, an optionally substituted C₁₋₄ alkoxy group or nitro group, respectively, or R^{1a} and R^{2a} may be bound to each other to form an optionally substituted C₂₋₆ alkylene or an optionally substituted methylenedioxy; the symbol * denotes the asymmetric center, or a salt thereof; optically active isomers of a compound represented by the formula (Ia'): 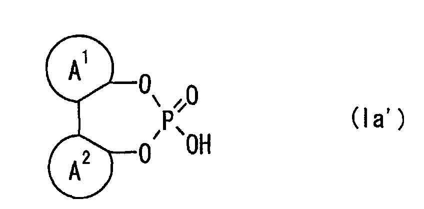 wherein A¹ ring and A² ring independently denote an optionally substituted aromatic ring, respectively or a salt thereof; and the like.

These compounds can easily be obtained according to the methods described in JP 61 - 103886 A, JP 55 - 47013 B, J. Org. Chem. 50, 4508(1985) and the like; and some of these compounds are commercially available. Specific examples of the compounds are 2-hydroxy-5,5-dimethyl-4-phenyl-1,3,2-dioxaphosphorinane-2-oxide, 4-(2-chlorophenyl)-2-hydroxy-5,5-dimethyl-1,3,2-dioxaphosphorinane-2-oxide, 4-(2,4-dichlorophenyl)-2-hydroxy-5,5-dimethyl-1,3,2-dioxaphosphorinane-2-oxide, 2-hydroxy-4-(2-methoxyphenyl)-5,5-dimethyl-1,3,2-dioxaphosphorinane-2-oxide, 2-hydroxy-4,4-dimethyl-5-phenyl-1,3,2-dioxaphosphorane-2-oxide, 2-hydroxy-5-(2-methoxyphenyl)-4,4-dimethyl-1,3,2-dioxaphosphorane-2-oxide, 1,1'-binaphthyl-2,2'-diyl hydrogenephosphate, 1,1'-binaphthyl-2,2'-diyl hydrogenephosphate, 3'-hydroxy-1,1'-binaphthyl-2,2'-diyl hydrogenephosphate, 9,9'-biphenanthryl-10,10'-diyl hydrogenephosphate, and the like.

Examples of the optically active sulfamic acid derivative include optically active isomers of a compound represented by the formula (Ib): 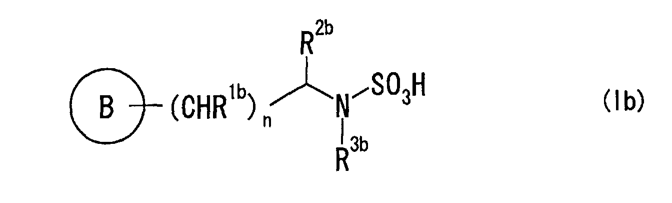 wherein B denotes an optionally substituted C₆₋₁₄ aryl group; R^{1b} denotes hydrogen atom or hydroxy; R^{2b} denotes a halogen atom, an optionally substituted hydrocarbon group, or optionally esterified carboxyl group; R^{3b} denotes a halogen atom or an optionally substituted hydrocarbon group; and n denotes 0 or 1, or a salt thereof; optically active isomers of a compound represented by the formula (Ic) : 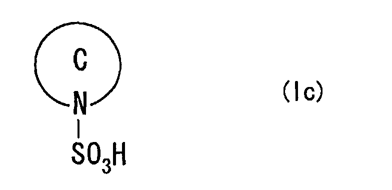 wherein C ring denotes pyrrolidine or piperidine respectively substituted with 1 or 2 substituents selected from optionally substituted hydrocarbon group, optionally esterified or amidated carboxyl group and cyano group, or a salt thereof; and the like.

These compounds are easily available as commercial products. Also, they can easily be produced from commercially available optically active amines, etc. according to the method described in J. Org. Chem. 23, 1133(1958) and the like. Further, likewise, they can be produced from commercially available racemic amines by optical resolution according to a known method after synthesis of racemic sulfamic acids. Specific examples of the compounds include 1-phenylethylsulfamic acid, 1-phenyl-2-(p-tolyl)ethylsulfamic acid, 1-(1-naphthyl)ethylsulfamic acid, 2-phenylpyrrolidine-1-sulfonic acid, 2-benzylpyrrolidine-1-sulfonic acid, 3-phenylpyrrolidine-1-sulfonic acid, 2-(4-methoxyphenyl)piperidine-1-sulfonic acid and the like.

Examples of the optically active sulfonic acid derivative include camphor-10-sulfonic acid, 3-bromocamphor-8-sulfonic acid, 1-phenylethanesulfonic acid, 1-phenylpropanesulfonic acid, and the like. These exemplified optically active sulfamic acid derivative can easily be obtained according to, for example, the method described in J. Org. Chem. 23, 1133(1958) and the like.

The aromatic ring of the "optionally substituted aromatic ring" represented by A¹ ring and A² ring in the above formula is, for example, benzene ring, naphthalene ring, phenanthrene ring and the like.

The compounds (Ia') are preferably optically active isomers of a compound represented by the formulas: 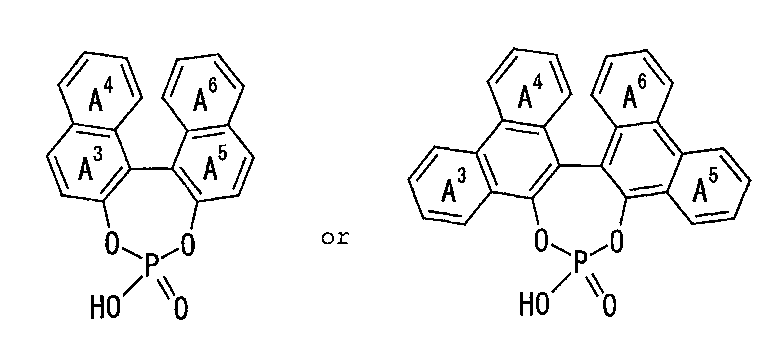 wherein A³ ring, A⁴ ring, A⁵ ring, and A⁶ ring denote an optionally substituted benzene ring, respectively, or a salt thereof.

In the formulas, A ring, A³ ring to A⁶ ring, and B may respectively have one or two substituents at any possible position and examples of the substitutes include an optionally substituted lower alkyl group, an optionally substituted hydroxy group, an optionally substituted thiol group, nitro group, an optionally substituted amino group, an acyl group, a halogen atom, cyano group, an optionally substituted methylenedioxy group (or adjacent two substituents are bound to each other) and the like.

As the optionally substituted lower alkyl group, in addition to a straight chain or branched chain unsubstituted C₁₋₆ alkyl group such as methyl, ethyl, n-propyl, isopropyl, n-butyl, isobutyl, sec-butyl, tert-butyl, pentyl, hextyl and the like, for example, there can be used these alkyl groups substituted with C₂₋₅ alkanoyl (e.g. acetyl, propionyl, and the like), carboxyl, C₁₋₄ alkoxycarbonyl (e.g., methoxycarbonyl, ethoxycarbonyl, butoxycarbonyl) and the like.

As the optionally substituted hydroxy group, in addition to unsubstituted hydroxy, for example, there can be used lower alkoxy groups (e.g., straight chain or branched chain C₁₋₆ alkoxy groups such as methyloxy, ethyloxy, n-propyloxy, isopropyloxy, n-butyloxy, isobutyloxy, sec-butyloxy, tert-butyloxy, pentyloxy, hexyloxy, etc.); lower alkanoyloxy groups (e.g., C₁₋₄ alkanoyloxy such as acetyloxy, propionyloxy, etc.); optionally substituted carbamoyloxy groups (e.g., in addition to unsubstituted carbamoyloxy, carbamoyloxy substituted with one or two C₁₋₄ alkyl groups such as methylcarbamoyloxy, ethylcarbamoyloxy, dimethylcarbamoyloxy, diethylcarbamoyloxy, methylethylcarbamoyloxy, etc.); and the like.

As examples of the optionally substituted thiol group, in addition to unsubstituted thiol group, for example, there can be used lower alkylthio groups (e.g., C₁₋₄ alkylthio groups such as methylthio, ethylthio, propylthio, etc.); lower alkanoylthio groups (e.g., C₁₋₄ alkanoylthio groups such as acetylthio, propionylthio, etc.); and the like.

As examples of the optionally substituted amino group, in addition to unsubstituted amino group, for example, there can be used lower alkylamino groups (e.g., C₁₋₄ alkylamino groups such as methylamino, ethylamino, propylamino, etc.); di-(lower alkyl)amino groups (e.g., di(C₁₋₄ alkyl)amino groups such as dimethylamino, diethylamino, etc.); C₁₋₄ alkanoylamino groups (such as acetamido, propionamido, etc.); and the like.

As examples of the acyl groups are, for example, there can be used alkanoyl groups (e.g., C₁₋₆ alkanoyl such as formyl; acetyl, propionyl, etc.); alkylsulfonyl groups (e.g., C₁₋₄ alkylsulfonyl such as methylsulfonyl, ethylsulfonyl, etc.); arylsulfonyl groups (e.g. benzenesulfonyl, p-toluenesulfonyl, etc.); optionally substituted carbamoyl groups (e.g. mono- or di-C₁₋₁₀ alkylcarbamoyl groups such as methylcarbamoyl, ethylcarbamoyl, dimethylcarbamoyl, diethylcarbamoyl. etc.; e.g., mono- or di-C₆₋₁₄ arylcarbamoyl groups such as phenylcarbamoyl, diphenylcarbamoyl, etc.; e.g., mono- or di-C₇₋₁₆ aralkyl-carbamoyl groups such as benzylcarbamoyl, dibenzylcarbamoyl, etc.); optionally substituted sulfamoyl groups (e.g. mono- or di-C₁₋₁₀ alkylsulfamoyl groups such as methylsulfamoyl, ethylsulfamoyl, dimethylsulfamoyl, diethylsulfamoyl, etc. ; e.g. mono- or di-C₆₋₁₄ arylsulfamoyl groups such as phenylsulfamoyl, diphenylsulfamoyl, etc.; e.g. mono- or di-C₇₋₁₆ aralkylsulfamoyl such as benzylsulfamoyl, dibenzylsulfamoyl, etc.) and the like.

Examples of the halogen atom include fluorine, chlorine, bromine, and iodine.

The optionally substituted methylenedioxy group is substituted at adjacent two carbon atoms of the benzene ring and examples thereof include, in addition to unsubstituted methylenedioxy group, methylene groups substituted with a halogen atom (e.g. fluorine atom, chlorine atom, bromine atom, iodine atom), nitro group, hydroxy group, amino group and the like.

A ring is preferably a benzene ring optionally substituted with one or two substituents selected from halogen, a C₁₋₆ alkyl group, a C₁₋₆ alkoxy group, . A ring is especially preferably that unsubstituted or substituted groups with one or two chlorine atoms and methoxy groups. The substituent is located at 2-, 4-, and 2,4-positions.

Substituents of A¹ and A⁶ are preferably one or two substituents selected from halogen atoms, hydroxy groups, C₁₋₆ alkoxy groups, C₁₋₆ alkyl groups, C₁₋₆ alkoxy-C₁₋₆ alkyl groups, C₆₋₁₄ aryl groups, and C₁₋₆ alkyl-carbonyl groups.

B is preferably a C₆₋₁₄ aryl group (phenyl group, naphthyl group) optionally substituted with one or two substituents selected from nitro, halogen atoms, C₁₋₆ alkyl groups and C₁₋₆ alkoxy groups.

C ring is pyrrolidine or piperidine optionally substituted with one or two substituents selected from hydrocarbon groups optionally substituted with one or two substituents selected from nitro, cyano, halogen atoms, hydroxy groups, C₁₋₄ alkoxy groups, carbonyl groups, carbamoyl groups, C₁₋₄ alkoxy-carbonylamino groups, and acylamino groups; carboxyl groups; C₁₋₄ alkoxy-carbonyl groups; carbamoyl groups and cyano groups, respectively.

As the optionally substituted hydrocarbon group of the substituent groups in R^{1a}, R^{2a}, R^{2b}, R^{3b} and C ring, in addition to unsubstituted C₁₋₄ alkyl such as methyl, ethyl, propyl, etc., for example, there are these alkyl groups substituted with one or two substituents selected from halogen atoms, nitro, cyano, C₁₋₄ alkanoyl (e.g., acetyl, propionyl and the like), carboxyl, hydroxy group, C₁₋₄ alkoxy (e.g., methoxy, ethoxy, propoxy and the like), C₁₋₄ alkoxy-carbonyl (e.g., methoxycarbonyl, ethoxycarbonyl, butoxycarbonyl and the like), carbamoyl, C₁₋₄ alkoxy-carbonylamino (e.g., methoxycarbonylamino, ethoxycarbonylamino, and the like), and the like.

As the optionally substituted C₁₋₄ alkoxy group, represented by R^{1a} and R^{2a}, in addition to unsubstituted C₁₋₄ alkoxy groups such as methyloxy, ethyloxy, etc., for example, there are these alkyl groups substituted with one or two substituents selected from C₁₋₄ alkanoyl (e.g., acetyl, propionyl and the like), carboxyl, hydroxy group, C₁₋₄ alkoxy (e.g., methoxy, ethoxy, propoxy and the like), and C₁₋₄ alkoxy-carbonyl (e.g., methoxycarbonyl, ethoxycarbonyl, butoxycarbonyl and the like), and the like.

Examples of the halogen atom represented by R^{1a} and R^{2a} include fluorine atom, chlorine atom, bromine atom, iodine atom and the like.

When R^{1a} and R^{2a} together form the optionally substituted alkylene group, examples of the optionally substituted alkylene group include, in addition to an unsubstituted alkylene group having 2 to 6 carbon atoms (dimethylene, trimethylene, tetramethylene, pentamethylene), these alkylene substituted at any position with one or two substituents selected from lower alkyl groups (e.g., C₁₋₄ alkyl such as methyl, ethyl, propyl, etc.), lower alkoxy groups (e.g., C₁₋₄ alkoxy such as methoxy, ethoxy, propoxy, etc.), hydroxy group, amino group, nitro group, halogen atoms (e.g., fluorine atom, chlorine atom, bromine atom, iodine atom), and the like.

Preferred examples of R^{1a} and R^{2a} include the case where both R^{1a} and R^{2a} are methyl groups and the case where both are bound each other to form tetramethylene group.

Examples of the "optionally esterified carboxyl group" represented by R^{2b}, and the "optionally esterified or amidated carboxyl group" of the substituent of C ring include the same groups as those defined with respect to the above-mentioned R₀ to R₁₀.

In case that the above-mentioned compound has an acidic group, it may form a salt with, for example, metals (e.g., sodium, potassium, calcium and the like) or ammonium ion and in case that the compound has a basic group, it may form an acid addition salt, for example, inorganic acid salts (e.g., hydrochloride, sulfate, hydrobromide, phosphate, and the like); organic acid salts (e.g., acetate, trifluoroacetate, succinate, maleate, fumalate, propionate, citrate, tartarate, lactate, oxalate, methanesulfonate, p-toluenesulfonate, and the like).

In the optical resolution in the production process of the present invention, the optical isomeric mixture of cyclic enaminone derivatives is dissolved in an inert organic solvent, which is appropriately selected from, for example, alcohols such as methanol, ethanol, 1-propanol, 2-propanol, 1-butanol; nitriles such as acetonitrile; amides such as N,N-dimethylformamide, N,N-dimethylacetamide; lactams such as N-methylpyrrolidone; ketones such as acetone, methyl-ethyl-ketone; esters such as ethyl acetate, methyl acetate; carboxylic acid such as formic acid, acetic acid; aromatic hydrocarbons such as toluene; ethers such as tetrahydrofuran, diisopropyl ether; halogenated hydrocarbons such as methylene chloride; aliphatic hydrocarbons such as hexane; and dimethyl sulfoxide; together with the above-mentioned optical resolution agent with a high acidity in an amount ranging from about 0.5 to 5 equivalent, preferably from about 0.5 to 2 equivalent, from about 0°C of reflux temperature of the solvent, preferably about 0 to 100°C. Then, after concentration if desired, a solvent appropriately selected among the above exemplified solvents is added and steps of heating, cooling or concentrating or combinations of these steps are carried out to precipitate a crystalline salt and then the crystals are isolated to complete the optical resolution.

The cooling temperature at the time of precipitating a salt is preferably in a range from about -20 to 20°C.

The concentration of the organic solvent may be carried out by distilling off the organic solvent under reduced pressure, for example, after dissolution of optically active cyclic enaminone derivative and an optical resolution agent in the organic solvent until a part or sufficient amount of a crystalline salt is precipitated.

Examples of the method for precipitating the crystalline salt include that by dissolving cyclic enaminone derivatives and an optical resolution agent in ethanol with heating and then adding dropwise ethyl acetate to effect crystallization.

The crystalline salt of the optically active cyclic enaminone derivative and the optical resolution agent obtained by the above-mentioned process may be subjected to recrystallization by a known method if necessary. Moreover, by treating with an alkali in an inert solvent if necessary, the desired optically active cyclic enaminone derivative or its salt can be obtained. Specifically, for example, the crystalline salt is distributed between an organic solvent such as ethyl acetate, tetrahydrofuran, methyl ethyl ketone, etc. and an aqueous alkali solution such as sodium hydroxide, etc. to obtain the free cyclic enaminone derivative. Thus-obtained optically active cyclic enaminone derivative may further be treated with an inorganic acid or an organic acid to isolate it in the form of the desired salt. Examples of the salt include hydrochloride, hydrobromide, sulfate, nitrate, phosphate, methanesulfonate, toluenesulfonate, 1-phenylethylsulfamate, and the like.

In the production process of the present invention, after the desired optically active cyclic enaminone derivative is isolated in the form of the salt of the optical resolution agent from an optically isomer mixture, the remaining other optically active cyclic enaminone derivative is subjected to hydrolysis to recover the 1,3-diketone derivative having a symmetry plane, the raw material compound. This hydrolysis treatment may be carried out according to a conventional acid hydrolysis reaction and, for example, the optically active cyclic enaminone derivative or its salt and about 0.1 to 100 equivalent, preferably about 0.1 to 50 equivalent, of an inorganic or organic acid selected from hydrochloric acid, sulfuric acid, nitric acid, phosphoric acid, hydrobromic acid, formic acid, acetic acid, trifluoroacetic acid, methanesulfonic acid, toluenesulfonic acid are stirred in water or a mixture of water and a water-soluble organic solvent properly selected from alcohols such as methanol, ethanol, etc. and ketones such as acetone, etc. at about 0 to 100°C for about 0.5 to 48 hours to quantitatively carry out the treatment.

The cyclic 1,3-diketone derivative having a symmetry plane to be used as a raw material in the production process of the present invention is per se well-known and easily available as a commercial product and from such a commercial product, it can easily obtained by conversion according to a known process.

More specifically, in the case of a compound having the partial structure (A) represented by the formula (A-2), the compound can easily be obtained according to a known method, for example, the process described in J. Am. Chem. Soc., 72. 1645 (1950) and further some other compounds are also easily available as commercial products. In the case of a compound having the partial structure (A) represented by the formula (A-1) or the formula (A-3), the compound can be easily produced from a commercially available compound, for example, 2,3-dimethylmeleic acid anhydride, 2,3-diphenylmaleic acid anhydride, 3,4,5,6-tetrahydrophthalic acid anhydride, 2,3-bis(2,4,5-trimethyl-3-thienyl)maleic acid anhydride and the like, by combination of known reactions.

The optically active cyclic enaminone derivatives obtained by the production process of the present invention are useful for synthetic raw materials or synthetic intermediates for producing optically active pharmaceuticals and agrochemicals.

For example, the optically active cyclic enaminone derivative represented by the following formula: 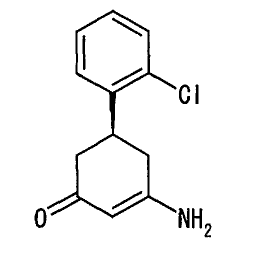 can be produced according to the same processes as those described in Example 1 to Example 4 hereinafter, and further the compound represented by the following formula: 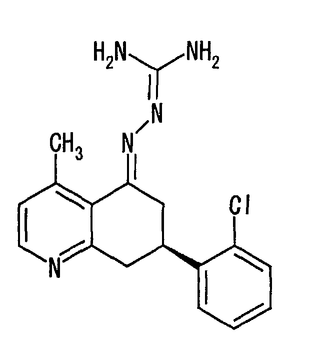 can be produced according to the same processes as those described in Reference Example 1 and Reference Example 2 hereinafter.

Also, for example, the optically active cyclic enaminone derivative represented by the following formula: 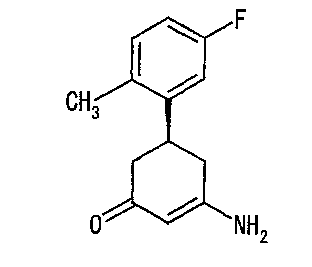 can be produced according to the same process as that described in Example 5 hereinafter, and further the compound represented by the following formula: 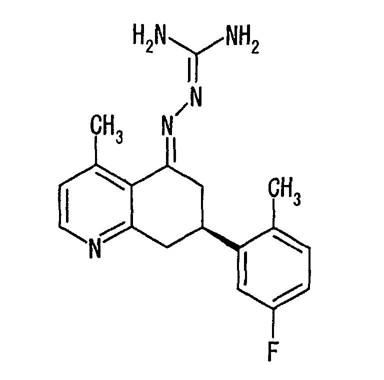 can be produced according to the same processes as those described in Reference Example 7 to Reference Example 9 hereinafter. The above-mentioned compound is useful as a Na⁺/H⁺ exchange inhibitor for treating or preventing ischemic cardiac disease, heart failure, and the like (cf. WO 99/42442).

### BEST MODE OF EMBODIMENTS OF THE INVENTION

The following Examples and Reference Examples further illustrate the present invention in detail but are not to be construed to limit the scope of the present invention.

### Examples

### Example 1

### Production of (±)-3-amino-5-(2-chlorophenyl)-2-cyclohexenon-1-one:

A mixture of 5-(2-chlorophenyl)cyclohexan-1,3-dione (180 mg), ammonium acetate (300 mg), and ethanol (3 ml) was stirred at 100°C for 2 hours. The resultant mixture was concentrated under reduced pressure and the resultant residue was mixed with water. The precipitated crystals thus obtained were collected by filtration, washed with water, and vacuum dried to obtain the titled compound (157 mg, yield 87.6 %).

¹HNMR (300 MHz, DMSO-d₆) δ: 2.23 (1H, dd J =15.8 Hz, 4.1 Hz), 2.30-2.70 (3H, m), 3.61 (1H, m), 5.05 (1H, s), 6.60-7.20 (2H, br), 7.20-7.55 (4H, m)

Chiral Fixed Phase HPLC Analysis (column: Daicel CHIRALCELO J-R, length 150 mm, inner diameter 4.6 mm; mobile phase: water/ethanol 65/35; flow rate 0.5 ml/min; detection wavelength 254 mm) (R)-isomer 49.7% (retention time 16.7 minutes), (S)-isomer 50.1% (retention time 19.1 minutes), completely racemic.

### Example 2

### Production of (S)-3-amino-5-(2-chlorophenyl)-2-cyclohexenon-1-one (S)-1-phenylethylsulfamate:

(±)-3-Amino-5-(2-chlorophenyl)-2-cyclohexenon-1-one (35.25 g) obtained by the above-mentioned Example 1 was dissolved in ethanol (71 ml) with heating and ethyl acetate (460 ml) was added dropwise over 1.5 hours to the resultant solution and stirred for 3 hours. The precipitated crystals were collected by filtration, washed with ice-cooled ethanol : ethyl acetate = 1 : 8 (140 ml), and vacuum dried to obtain the titled compound (23.5 g). The crude crystals (23 g) thus obtained were dissolved in ethanol (92 ml) with heating and ethyl acetate (230 ml) was added dropwise over about 1 hour to the resulting solution and stirred for 3 hours. The precipitated crystals were collected by filtration, washed with ice-cooled ethanol : ethyl acetate = 1 : 8 (92 ml), and air dried to obtain the titled compound (14 g, yield 20.8%).

¹HNMR (300 MHz, DMSO-d₆) δ: 1.55 (3H, d, J = 6.8 Hz, 4.1 Hz), 2.28 (1H, dd J =15.9 Hz, 4.0 Hz), 2.24-2.75 (3H, m), 3.63 (1H, m), 4.49 (1H, q, J = 6.8 Hz), 5.15 (1H, s), 6.90-7.60 (2H, br), 7.20-7.60 (4H, m)

### Example 3

### Production of (S)-3-amino-5-(2-chlorophenyl)-2-cyclohexenon-1-one:

A mixture of the compound (1 g) obtained in Example 2, a mixed solvent (20 ml) of ethyl acetate and methyl ethyl ketone (1 : 1), and an aqueous 1 N sodium hydroxide solution (20 ml) was stirred at a room temperature for 1 hour. After confirmation of complete dissolution of the solids, the organic phase was washed with saturated salt water (20 ml) and dried over magnesium sulfate (lg). The solvent was distilled off under reduced pressure and the resultant powder was vacuum dried to obtain the titled compound (0.52 g, yield 99.2 %).

¹HNMR (300 MHz, DMSO-d₆) δ: 2.23 (1H, dd J =15.8 Hz, 4.1 Hz), 2.30-2.70 (3H, m), 3.61 (1H, m), 5.05 (1H, s), 6.60-7.20 (2H, br), 7.20-7.55 (4H, m)

Chiral Fixed Phase HPLC Analysis (column: Daicel CHIRALCELO J-R, length 150 mm, inner diameter 4.6 mm; mobile phase: water/ethanol 65/35; flow rate 0.5 ml/min; detection wavelength 254 mm) (S) -isomer 99.6% (retention time 18.8 minutes), (R)-isomer 0.3% (retention time 17.1 minutes), optical purity 99.4% ee.

### Example 4

### Production of 5-(2-chlorophenyl)-2-cyclohexan-1,3-dione:

Using (R)-1-phenylethylsulfamic acid, (R)-3-amino-5-(2-chlorophenyl)-2-cyclohexenone (200 mg, 99.2 ee) obtained according to the same manner as that of Example 1 to Example 3 and 0.5 M sulfuric acid (10 ml) were mixed and stirred at 100°C for 2 hours. After cooling, the precipitated crystals were collected by filtration and subjected to washing with water and vacuum drying to obtain the titled compound (187 mg, yield 93.1 %).

¹HNMR (300 MHz, DMSO-d₆) δ: 2.30-2.75 (4H, m), 3.70 (1H, m), 5.32 (1H, s), 7.15-7.60 (4H, m), 10.80-11.6 (1H, br)

### Example 5

### Production of (-)-3-amino-5-(5-fluoro-2-methylphenyl)-2-cyclohexenon-1-one:

A mixture of (±)-3-amino-5-(5-fluoro-2-methylphenyl)-2-cyclohexenon-1-one (2.82 g), (-)-4-(2,4-dichlorophenyl)-2-hydroxy-5,5-dimethyl-1,3,2-dioxaphosphrinane-2-oxide (4.0 g), and acetonitrile (42.3 ml) was refluxed with heating for 15 minutes. After stirring for 2 hours at room temperature, the precipitated crystals were collected by filtration and washed with acetonitrile (20 ml). The crystals thus obtained were suspended in tetrahydrofuran (36 ml) and dissolved by adding dropwise alcohol (9.43 ml) under reflux with heating. After stirring at 5°C for 2 hours, the precipitated crystals were collected by filtration and washed with tetrahydrofuran-isopropyl ether (5 : 1) to obtain a diastereomer salt (2.89 g, 43.5%) of (-)-3-amino-5-(5-fluoro-2-methylphenyl)-2-cyclohexenon-1-one and (-)-4-(2,4-dichlorophenyl)-2-hydroxy-5,5-dimethyl-1,3,2-dioxaphosphrinane-2-oxide. To the mixture of the diastereomer salt (2.8 g), toluene (5.6 ml) and water (28 ml), 2N NaOH (5.85 ml) were added at room temperature and stirred at 5°C for 2 hours. The precipitated crystals was collected by filtration and washed to obtain the title compound (1.00 g. 36.6%).

¹HNMR (CDCl₃) δ: 2.30 (3H, s), 2.20-2.75 (4H, m), 3.47-3.65 (1H, m), 4.75 (2Hb, s), 5.35(1H, s), 6.80-7.03 (2H, m), 7.08-7.19 (1H, m)

Chiral Fixed Phase HPLC Analysis (column: Daicel CHIRALCELO J-R, length 150 mm, inner diameter 4.6 mm; mobile phase: 0.2MnaClO₄/acetonitrile 85/15 fabricated by HClO₄ to pH2; flow rate 1.0 ml/min; detection wavelength 254 mm) (-)-isomer (retention time 24.0 minutes), (+)-isomer (retention time 22.2 minutes), optical purity 92% ee (HPLC).

### Example 6

### Production of (-)-3-amino-5-(5-fluoro-2-methylphenyl)-2-cyclohexen-1-one and (S)-4-(2-chlorophenyl)-2-hydroxy-5,5-dimethyl-1,3,2-dioxaphosphrinane-2-oxide salt

Racemic (-)-3-amino-5-(5-fluoro-2-methylphenyl)-2-cyclohexen-1-one 50 mg and (S)-4-(2-chlorophenyl)-2-hydroxy-5,5-dimethyl-1,3,2-dioxaphosphrinane-2-oxide 63.1 mg were dissolved in 0.3 ml of isopropyl alcohol and 0.6 ml of acetonitrile and allowed to stand overnight in a refrigerator. Next day, the precipitated crystals were collected by filtration to obtain crystals 63 mg. The results of Chiral Fixed Phase HPLC Analysis (column: CHIRALPAK AD (manufactured by Daicel Chem. Ind., Ltd.); mobile phase: hexane/isopropyl alcohol/diethylamine 90:10:0.1; flow rate 0.6 ml/min; detection: UV 280 mm; temperature: at room temperature) showed the excess ratio of diastereomer was 62% de. Sixty-two mg of the obtained compound was recrystallized using 1 ml of tetrahydrofuran. The crystals thus obtained were 27.6 mg (yield 25%). The results of HPLC analysis showed the excess ratio of diastereomer was 98% de.

### Example 7

### Production of (-)-3-amino-5-(5-fluoro-2-methylphenyl)-2-cyclohexen-1-one and (S)-(-)-2-hydroxy-4-(2-methoxyphenyl)-5,5-dimethyl-1,3,2-dioxaphosphrinane-2-oxide salt.

Racemic (-)-3-amino-5-(5-fluoro-2-methylphenyl)-2-cyclohexen-1-one 50 mg and (S)-(-)-2-hydroxy-4-(2-methoxyphenyl)-5,5-dimethyl-1,3,2-dioxaphosphrinane-2-oxide 62.1 mg were dissolved in 0.1 ml of isopropyl alcohol and 0.6 ml of acetonitrile and allowed to stand overnight in a refrigerator. Next day, the precipitated crystals were collected by filtration to obtain crystals 66mg. The results of Chiral Fixed Phase HPLC Analysis (column: CHIRALPAK AD (manufactured by Daicel Chem. Ind., Ltd.); mobile phase: hexane/isopropyl alcohol/diethylamine 90:10:0.1; flow rate 0.6 ml/min; detection: UV 280 mm; temperature: at room temperature) showed the excess ratio of diastereomer was 56% de. Sixty-five mg of the obtained compound was suspended in 1.5 ml of tetrahydrofuran and 0.5 ml of isopropyl alcohol and allowed to stand overnight in a refrigerator. Next day, the precipitated crystals were collected by filtration to obtain crystals 40.9mg (yield 37%). The results of HPLC analysis showed the excess ratio of diastereomer was 86% de.

### Reference Example 1

### Production of (S)-(-)-7-(2-chlorophenyl)-5-guanidinoimino-4-methyl-5,6,7,8-tetrahydroquinoline:

The compound (12 g) obtained in Example 3 was dissolved in ethanol (120 ml) and mixed with toluene (360 ml), 1,1-dimethoxy-3-butanone (7.2 g), and sodium methoxide (3.7 g) and refluxed with heating for 2 hours. After cooling to room temperature, the resultant mixture was mixed with water (60 ml) and stirred and after that, an organic layer was separated. After the water layer was washed with toluene (60 ml), the organic layer was added and washed with saturated salt water (50 ml) and then concentrated under reduced pressure. The residue thus obtained was dissolved in ethanol (130 ml) and mixed with aminoguanidine hydrochloride (3.23 g) and concentrated hydrochloric acid (8.5 ml) and refluxed with heating for 3.5 hours. The residue thus obtained from the reaction solution by concentration under reduced pressure was dissolved in water (100 ml) and adjusted to pH 9 with an aqueous 8M sodium hydroxide and then extracted with 2-butanone (165 ml, 65 ml). All of the organic layers obtained were mixed and washed with saturated salt water (65 ml) and then the solvent was removed by vacuum distillation. The residue obtained was dissolved in ethanol (10 ml) and when the crystals were precipitated, ethanol (10 ml) was further added and diisopropyl ether (20 ml) was added dropwise thereto. After the resultant mixture was stirred at room temperature for 1 hour and then for 1 hour with ice-cooling, the crystals were collected by filtration and washed with ice-cooled ethanol : diisopropyl ether = 1 : 4 (50 ml) and vacuum-dried to obtain the titled compound (6.66 g, yield 73.2 %).

¹HNMR (300 MHz, CDCl₃) δ: 2.60-280 (1H, m), 2.68 (3H, s), 3.05-3.30 (2H, m), 3.50-3.70 (2H, m), 7.05 (1H, d, J= 5.0 Hz), 7.10-7.40 (4H, m), 8.26 (1H, d, J = 5.0 Hz)

### Reference Example 2

### Production of (S)-(-)-7-(2-chlorophenyl)-5-guanidinoimino-4-methyl-5,6,7,8-tetrahydroquinoline dimethanesulfonate:

The compound (6.5 g) obtained in Reference Example 1 was dissolved in ethanol (42 ml) with heating to separate impurities by filtration and then, mixed with methanesulfonic acid (3.8 g) and stirred at a room temperature for 2 hours. The precipitated crystals were collected by filtration, washed with ethanol : acetone = 1 : 1 (50 ml) and vacuum dried to obtain the titled compound (7.44 g, yield 72.2 %).

¹HNMR (300 MHz, DMSO-d₆) δ: 2.38 (6H, s), 2.79 (1H, dd, J = 17.4 Hz, 11.7 Hz), 2.89 (3H, s), 3.05-3.30 (2H, m), 3.45-3.80 (2H, m), 7.30-7.55 (3H, m), 7.55-7.70 (1H, m), 7.87 (1H, d, J = 6.0 Hz), 8.67 (1H, d, J = 6.0Hz)

Chiral Fixed Phase HPLC Analysis (column: ULTRONES-OVM Shinwa Kagaku, length 150 mm, inner diameter 4.6 mm; mobile phase: 0.02 M KH₂PO₄ /ethanol 77/23; flow rate 1.0 ml/min; detection wavelength 254 mm) (S)-isomer 99.5% (retention time 6.2 minutes), (R)-isomer under detection limit, optical purity 100% ee.

### Reference Example 3

### Production of 5-fluoro-2-methylbenzaldehyde:

Iodine (about 1 mg) was added to a mixture of magnesium (26.28 g) and tetrahydrofuran (600 ml) and 2-bromo-4-fluorotoluene (200.4 g) was added dropwise thereto with ice-cooling (the inner temperature was kept at 65°C or lower). After completion of the dropwise addition, the mixture was stirred for 1 hour to prepare a Grignard reagent. Then, N-formylmorpholine (134.3 g) was added dropwise thereto with ice-cooling. After stirring at room temperature for 1 hour, 6 N HCl (400 ml) was added dropwise and extracted with ethyl acetate. The extract was successively washed with 5% salt water and water and then the solvent was removed by distillation to quantitatively obtain the titled compound.

¹HNMR (CDCl₃) δ : 2.64 (3H, s), 7.18-7.53 (2H, m), 7.47-7.53 (1H, m), 10.25 (1H, d, J = 1.8 Hz)

### Reference Example 4

### Production of 4-(5-fluoro-2-methylphenyl)3-buten-2-one:

A solution of 5-fluoro-2-methylbenzaldehyde (100 g) in acetone (136 ml) was added dropwise to a mixture of a solution of sodium hydroxide (30.35 g) in water (815 ml) and acetone (543 ml) over about 2 minutes. After stirring at 30°C for 20 minutes, acetone was removed by distillation under reduced pressure and extracted with ethyl acetate. The extract was successively washed with 5% salt water and water and then the solvent was removed by distillation to obtain the titled compound (122.97 g, 95.3%).

¹HNMR (CDCl₃) δ: 2.39 (3H, s), 2.41 (3H, s), 6.63 (1H, d, J =16 Hz), 6.94-7.27 (2H, m), 7.74 (1H, dd, J = 1.4, 16 Hz).

### Reference Example 5

### Production of 5-(5-fluoro-2-methylphenyl)cyclohexan-1,3-dione:

20% Sodium ethoxide (113.81 g) was added dropwise to a solution of 4-(5-fluoro-2-methylphenyl)-3-buten-2-one (50g) and diethyl malonate (53.57 g) in ethanol (100 ml) at 10°C or lower. The mixture was stirred at room temperature for 30 minutes and then 2 hours with refluxing. Then, the solvent was removed by distillation. To the residue was added 2N NaOH (154 ml) and the mixture was stirred at 90°C for 2.5 hours. To the mixture were added successively 2.5 M H₂SO₄ at room temperature and further toluene (100 ml), and the resultant mixture was stirred at 70°C for 30 minutes and further stirred for 1 hour with refluxing. After ice-cooling, precipitated crystals were collected by filtration to obtain the titled compound (39.28 g, 63.6%).

¹HNMR (CDCl₃) δ: 2.30 (3H, s), 2.27-2.56 (4H, m), 2.5-4.4 (1H, b), 3.44-3.63 (1H, m), 5.55 (1H, s), 6.77-7.01 (2H, m), 7.09-7.17 (1H, m)

### Reference Example 6

### Production of (±)-3-amino-5-(5-fluoro-2-methylphenyl)-2-cyclohexenon-1-one:

A mixture of 5-(5-fluoro-2-methylphenyl)cyclohexan-1,3-dione (34 g), ammonium acetate (35.22 g), and isopropanol (340 ml) was stirred for 2.5 hours with refluxing and then the solvent was removed by distillation. To the residue were added tetrahydrofuran (90 ml) and then isopropyl ether (450 ml) at 57°C to effect crystallization. After stirring at 5°C for 1 hour, the crystals were collected by filtration and washed with tetrahydrofuran-isopropyl ether (1 : 5) to obtain the titled compound (31.2 g, 92.2%).

¹HNMR (CDCl₃) δ: 2.30 (3H, s), 2.20-2.75 (4H, m), 3.47-3.65 (1H, m), 4.75 (2Hbs), 5.35 (1H, s), 6.80-7.03 (2H, m), 7.08-7.19 (1H, m)

### Reference Example 7

### Production of (-)-7-(5-fluoro-2-methylphenyl)-4-methyl-7,8-dihydroquinolin-5(6H)-one:

To a mixture of (-)-3-amino-5-(5-fluoro-2-methylphenyl)-2-cyclohexenon-1-one (0.95 g), 1,1-dimethoxy-3-butanone (1.15 g), and toluene (9.5 ml) were added potassium carbonate (0.12 g) and 28% sodium methoxide (1.0 g) and the mixture was stirred at 71 to 73°C for 2 hours. Further, 28% sodium methoxide (0.1 g) was added and the mixture was stirred at the same temperature for 2 hours and after the mixture was cooled to room temperature, water (6 ml) was added. The toluene layer was separated and washed with water and then the solvent was removed by distillation to obtain the titled compound (1.15 g, 98.8%).

¹HNMR (CDCl₃) δ: 2.33 (3H, s), 2.78-2.98 (2H, m), 3.24 (1H, dd, J = 11.16 Hz), 3.28-3.44 (1H, m), 3.55-3.74(1H, m), 6.82-7.04 (2H, m), 7.12 (1H, d, J = 5 Hz), 7.07-7.22 (2H, m), 8.50 (1H, d, J = 5Hz)

### Reference Example 8

### Production of (5E, 7S)-[7-(5-fluoro-2-methylphenyl)-4-methyl-7,8-dihydro-5(6H)-quinolinyldeneamino]guanidine di-hydrochloride:

To a solution of (-)-7-(5-fluoro-2-methylphenyl)-4-methyl-7,8-dihydroquinolin-5(6H)-one (1.15 g) in methanol (11.7 ml) was added aminoguranidine hydrochloride (0.72 g) and the mixture was stirred at 65°C for 20 minutes. After that, concentrated hydrochloric acid (1.15 ml) was further added and refluxed with heating for 2 hours. The solvent was removed by distillation and the residue was crystallized in isopropanol to obtain the titled compound (1.68 g, 100%).

¹HNMR (DMSO-d₆) δ: 2.31 (3H, s), 2.72-3.03 (1H, m), 2.90 (3H, s), 3.13-3.57 (4H, m), 6.93-7.06 (1H, m), 7.17-7.40 (2H, m), 7.50-8.40 (4H, br), 7.85 (1H, d, J = 6 Hz), 8.65 (1H, d, J- 6 Hz), 11.39 (1H, s)

### Reference Example 9

### Production of (5E, 7S)-[7-(5-fluoro-2-methylphenyl)-4-methyl-7,8-dihydro-5(6H)-quinolinyldenamino]guanidine dimethanesulfonate:

To a solution of (5E, 7S)-[7-(5-fluoro-2-methylphenyl)-4-methyl-7,8-dihydro-5(6H)-quinolinyldeneamino]guanidine di-hydrochloride (1.68 g) in methanol (11.5 ml) was added dropwise 28% sodium methoxide (2.51 g). After stirring for 20 minutes, water (4.1 ml) was added and the solvent was removed under reduced pressure. Water (1.17 ml) and ethyl acetate (3.5 ml) were added to the residue to dissolve it. Then, heptane (10. 5 ml) was added dropwise thereto to effect crystallization. After stirring at 5°C for 1 hour, the crystals were collected by filtration and washed with ethyl acetate-heptane (1 : 3) to obtain (5E, 7S)-[7-(5-fluoro-2-methylphenyl)-4-methyl-7,8-dihydro-5(6H)-quinolinyldeneamino]guanidine (1.15 g, 81.7%). Methanesulfonic acid (0.71 g) was added dropwise to a solution (8.05 ml) of this compound (1.15 g) in ethanol (8.05 ml) at room temperature. After cooling to 5°C and stirring for 2 hours, the precipitated crystals were collected by filtration to obtain the titled compound (1.40 g, 76.5%).

¹HNMR (DMSO-d₆) δ: 2.30 (3H, s), 2.35 (6H, s), 2.58-2.95 (1H, m), 2.86 (3H, s), 3.00-3.23 (2H, m), 3.44-3.60 (2H, m), 6.96-7.09 (1H, m), 7.20-8.40 (4H, br), 7.81 (1H, d, J = 5Hz), 8.65 (1H, d, J =6 Hz), 10.68 (1H, s)

Chiral Fixed Phase HPLC analysis (the same conditions as those in Example 1) optical purity 100 % ee.

### INDUSTRIAL APPLICABILITY

According to the production process of the present invention, an optically active cyclic enaminone derivative which is useful as a synthetic raw material for stereospecific pharmaceuticals and agrochemicals and the like can be produced highly efficiently without consuming in vain its precursor, a cyclic 1,3-diketone derivative having a symmetry plane.

## Claims

1. A process for producing an optically active cyclic enaminone derivative comprising optically resolving an optical isomeric mixture of cyclic enaminone derivatives using an optical resolution agent with a high acidity.

2. A process for producing an optically active cyclic enaminone derivative comprising aminating one carbonyl group of the diketone of a cyclic 1,3-diketone derivative having a symmetric plane to obtain an optically isomeric mixture of chiral cyclic enaminone derivatives and subjecting the mixture to optical resolution.

3. A process for producing an optically active cyclic enaminone derivative comprising using a cyclic 1,3-diketone derivative having a symmetry plane as a raw material, aminating one carbonyl group of the diketone of the derivative to obtain an optically isomeric mixture of chiral cyclic enaminone derivatives, subjecting the mixture to optical resolution to obtain one optically active cyclic enaminone derivative, and hydrolyzing the other optically active cyclic enaminone derivative to recover the raw material, the cyclic 1,3-diketone derivative having a symmetry plane.

4. The process according to claim 2 or 3, wherein the cyclic 1,3-diketone derivative having a symmetry plane has a 5- to 7-membered ring structure.

5. The process according to claim 2 or 3, wherein the cyclic 1,3-diketone derivative having a symmetry plane is represented by the following formula (I): 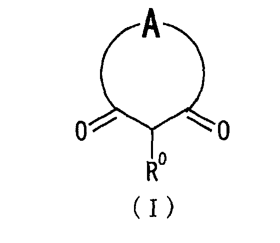 and the partial structure (A) of the formula (I): 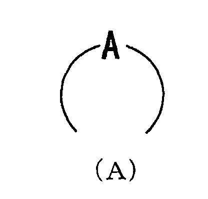 is represented by one of the following formulas (A-1) to (A-3): 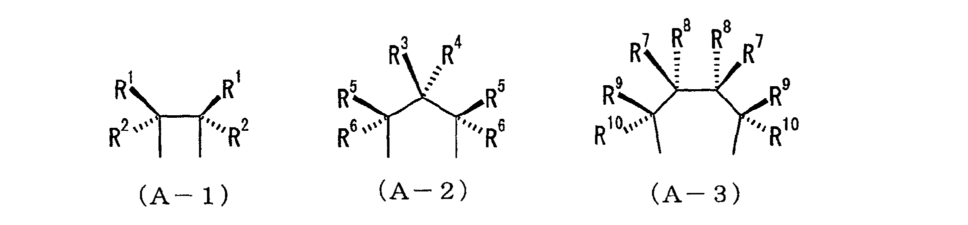 wherein R⁰ to R¹⁰ independently denote hydrogen atom or a substituent (provided that R¹ and R² do not denote the same group; R³ and R⁴ as well as R⁵ and R⁶, respectively, do not denote the same group simultaneously; and R⁷ and R⁸ as well as R⁹ and R¹⁰, respectively, do not denote the same group simultaneously).

6. The process according to claim 5, wherein the partial structure (A) is represented by the following formula (A-1): 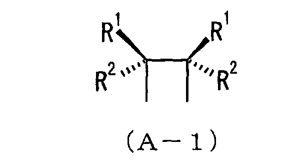 wherein R¹ and R² are as defined above.

7. The process according to claim 5, wherein said partial structure (A) is represented by the following formula (A-2): 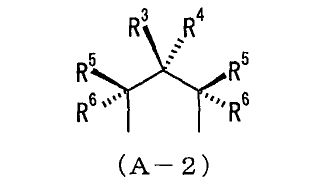 wherein R³, R⁴, R⁵ and R⁶ are as defined above.

8. The process according to claim 7, wherein R³ and R⁴ of said compound represented by the formula (A-2) of said partial structure (A) independently denote hydrogen atom or an optionally substituted 5- or 6-membered aromatic homocyclic or heterocyclic group, respectively; R⁵ and R⁶ independently denote hydrogen atom, a halogen atom, an optionally substituted straight or branched chain aliphatic hydrocarbon group, an optionally substituted hydroxy group, or an optionally substituted mercapto group (provided that R³ and R⁴ as well as R⁵ and R⁶, respectively, do not denote the same group simultaneously).

9. The process according to claim 7, wherein R³ and R⁴ of said compound represented by the formula (A-2) of said partial structure (A) independently denote hydrogen atom or an optionally substituted 5- or 6-membered aromatic homocyclic or heterocyclic group, respectively, (provided that R² and R³ do not denote the same group); R⁵ and R⁶ denote hydrogen atom.

10. The process according to claim 5, wherein said partial structure (A) is represented by the following formula (A-3): 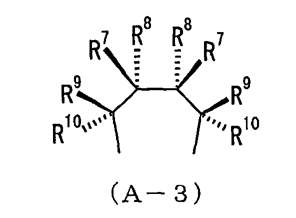 wherein R⁷ to R¹⁰ are as defined above.

11. The process according to claim 2 or 3, wherein the optical resolution is carried out using an optical resolution agent with a high acidity.

12. The process according to claim 1 or 11, wherein the optical resolution agent is an optically active sulfonic acid derivative or sulfamic acid derivative.

13. The process according to claim 1 or 11, wherein the optical resolution agent is an optically active phosphoric acid derivative.

14. An optically active cyclic enaminone derivative represented by the following formula: 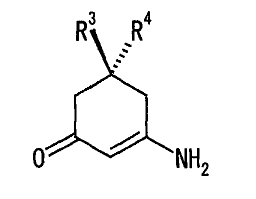 wherein R³ and R⁴ independently denote hydrogen atom or an optionally substituted 5- or 6-membered aromatic homocyclic or heterocyclic group, respectively (provided that R³ and R⁴ do not denote the same group).

15. A process for producing (-)-3-amino-5-(5-fluoro-2-methylphenyl)-2-cyclohexen-1-one comprising optically resolving (±)-3-amino-5-(5-fluoro-2-methylphenyl)-2-cyclohexen-1-one using (-)-4-(mono- or di-chloro or methoxypheny)-2-hydroxy-5,5-dimethyl-1,3,2-dioxaphosphorinane-2-oxide.

16. A process for producing (5E, 7S)-[7-(5-fluoro-2-methylphenyl)-4-methyl-7,8-dihydro-5(6H)-quinolinylideneamino]guanidine or its salt comprising reacting (-)-7-(5-fluoro-2-methylphenyl)-4-methyl-7,8-dihydroquinolin-5(6H)-one, which is obtained by reacting 1,1-dimethoxy-3-butanone with (-)-3-amino-5-(5-fluoro-2-methylphenyl)-2-cyclohexenon-1-one obtained by optically resolving (+)-3-amino-5-(5-fluoro-2-methylphenyl)-2-cyclohexenon-1-one using (-)-4-(mono- or di-chloro or methoxypheny)-2-hydroxy-5,5-dimethyl-1,3,2-dioxaphosphorinane-2-oxide, with aminoguanidine or its salt.
